# EUROPEAN PATENT APPLICATION

(11) **EP 4 303 224 A1**
(43) Date of publication of application: **10.01.2024**
(21) Application number: 22763297.3
(22) Date of filing: 02.03.2022
(51) Int. Cl.: C07K 1/02

(54) **PROTEIN FOLDING AGENT**

(30) Priority: 03.03.2021 JP 2021033583; 14.02.2022 JP 2022020271
(71) Applicant: Tohoku University, Sendai-shi, Miyagi 980-8577 (JP)
(72) Inventor: MURAOKA Takahiro, Fuchu-shi, Tokyo 183-8538 (JP); OKADA Shunsuke, Fuchu-shi, Tokyo 183-8538 (JP); MATSUMOTO Yosuke, Fuchu-shi, Tokyo 183-8538 (JP); OKUMURA Masaki, Sendai-shi, Miyagi 980-8577 (JP); INABA Kenji, Sendai-shi, Miyagi 980-8577 (JP); MATSUSAKI Motonori, Sendai-shi, Miyagi 980-8577 (JP)
(74) Representative: Mewburn Ellis LLP
(86) International application number: PCT/JP2022/008737
(87) International publication number: WO 2022/186243

(57) **Abstract**

Provided is a protein folding agent capable of folding a protein with high efficiency. A protein folding agent contains: as an active ingredient, at least one selected from compounds represented by formulas (1) to (14), salts thereof, and solvates thereof.

## Description

### Technical Field

The present invention relates to a protein folding agent.

### Background Art

Proteins are materials that are widely used in industry, mainly in preparations. Since formation of a native structure is essential for proteins to exert individual functions and activities, an efficient folding technique to a native structure is directly linked to and important in cost reduction or the like in industrial use of proteins. Accordingly, an oxidative folding accelerator to a native structure is useful for large-scale production of a protein preparation. For example, in an oxidative folding process for an important protein preparation such as insulin or immunoglobulin, native structure formation accompanied by introduction of a native disulfide bond is a rate determining step reaction.

A typical control method for an oxidative folding process is shuffling of a disulfide bond. In this method, a reducing agent and an oxidizing agent are allowed to coexist in the folding process, and formation and cleavage of the disulfide bond are repeatedly caused, thereby introducing a most stable native structure. Typical reducing agent and oxidizing agent used in this case are glutathione (GSH) and β-mercaptoethanol (β-ME), and dithiothreitol (DTT) and oxidized glutathione (GSSG), respectively. For example, in a case of RNase A, recovery yields in presence of GSH/GSSG and in absence of GSH/GSSG (in air) are compared, and in the presence of GSH/GSSG, an enzyme activity at the same time in an initial stage of folding is about twice higher, and a rate of activity recovery (1/2 activity recovery time) is about 2.8 times rapider (NPL 1). Further, as a small molecular reducing agent (and an oxidizing agent to be combined therewith) used in oxidative folding, GSH/GSSG (NPL 1), GSH + (1)-trans-1,2-bis(2-mercaptoacetamido)cyclohexane (BMC)/GSSG (NPL 2), aromatic thiols and their disulfides (NPL 3), cyclic selenoxide (NPL 4), a peptide having a Cys-XX-Cys structure (X is any amino acid, and Cys is cysteine) (NPL 5), seleno glutathione (NPL 6), and a selenol-containing peptide (NPL 7) are known. However, cyclic selenoxide and seleno glutathione have a toxicity problem due to use of heavy metal selenium. Further, since BMC is used as an auxiliary agent for GSH, there is a problem that it is needed to add a large amount of additives as compared with a GSH/GSSG system. Since the aromatic thiols are substances having benzene rings, there is a problem that non-specific adsorption to proteins due to hydrophobic interaction may occur. The peptide having a Cys-XX-Cys structure has problems of structural unstability such as decomposition due to protease contamination and high cost for synthesis thereof. Further, low-molecular-weight thiols such as cysteamine have a problem of strong fetor associated with high volatility. Further, when these existing compounds are used, an effect of suppressing aggregation of oxidative folding intermediates is low, which is a factor lowering a yield.

Accordingly, a protein folding agent capable of folding a protein with high efficiency while avoiding problems in the related art is desired.

### Citation List

### Non Patent Literature

NPL 1: A. K. Ahmed et al., J. Biol. Chem., 1975, 250, 8477-8482
NPL 2: K. J. Woycechowsky et al., Chem. Biol., 1999, 6, 871-879
NPL 3: D. J. Madar et al., J. Biotech., 2009, 142, 214-219
NPL 4: K. Arai, K et al., Chem. Eur. J., 2011, 17, 481-485
NPL 5: W. J. Lees et al., Curr. Opin. Chem. Biol., 2008, 12, 740-745
NPL 6: J. Beld et al., Biochemistry. 2007 May 8;46(18) :5382-90
NPL 7: S. Tsukagoshi et al., Chem. Asian J. 2020 September 1;15(17):2646-52

### Summary of Invention

### Technical Problem

Therefore, an object of the invention is to provide a protein folding agent capable of folding a protein with high efficiency.

### Solution to Problem

As a result of various studies on methods for solving the above problems, the present inventors have found that by using a compound having a specific structure, a salt thereof, and a solvate thereof, a disulfide bond can be introduced into a protein with high efficiency, that is, at a high yield and/or at a high speed, and have completed the invention.

That is, the gist of the invention is as follows.
[1] A protein folding agent containing: as an active ingredient, at least one selected from compounds represented by the following formulas, salts thereof, and solvates thereof, 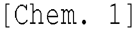 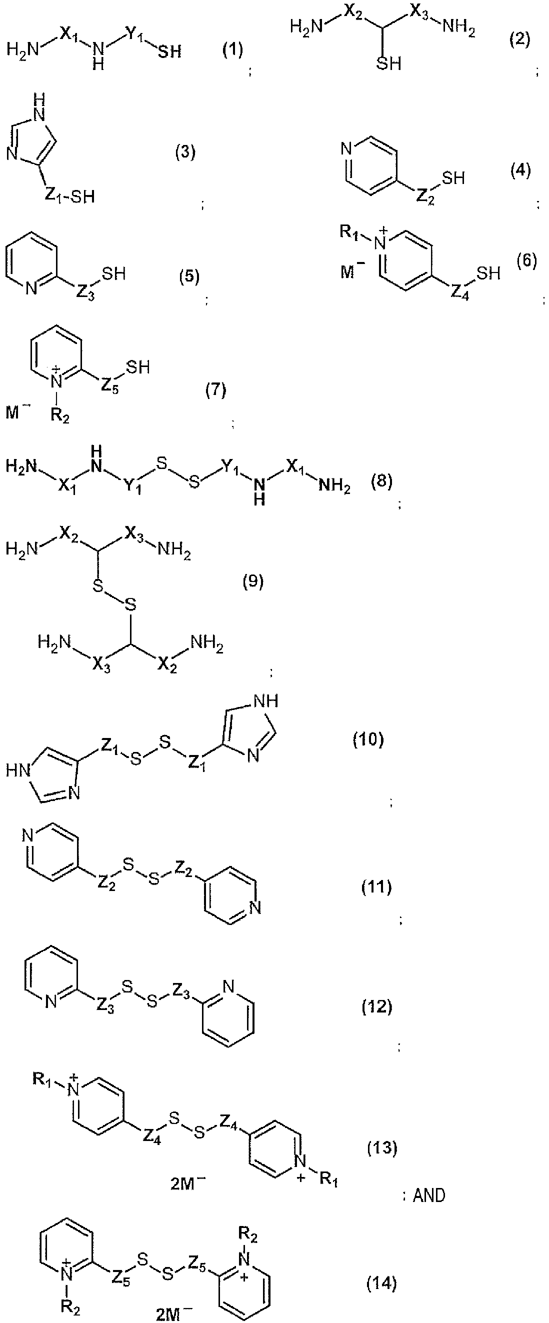 [in the formulas,
   X₁, X₂, and X₃ are each independently an alkylene group having 1 to 10 carbon atoms which may contain 1 to 5 oxygen atoms in a molecular chain,
   Y₁'s are each independently an alkylene group having 1 to 10 carbon atoms which may contain 1 to 5 oxygen atoms in a molecular chain,
   Z₁, Z₂, Z₃, Z₄, and Z₅ are each independently an alkylene group having 1 to 10 carbon atoms which may contain 1 to 5 oxygen atoms in a molecular chain,
   R₁ and R₂ are each independently an alkyl group having 1 to 24 carbon atoms, and
   M⁻'s are each independently a chlorine ion, a bromine ion, or an iodide ion].
[2] The protein folding agent according to [1], in which at least one selected from the compounds represented by the formulas (1) to (7), the salts thereof, and the solvates thereof is contained as the active ingredient.
[3] The protein folding agent according to [1], in which at least one selected from the compounds represented by the formulas (8) to (14), the salts thereof, and the solvates thereof is contained as the active ingredient.
[4] The protein folding agent according to [1], in which at least one selected from: the compounds represented by the formulas (1) to (7), the salts thereof, and the solvates thereof; and at least one selected from compounds represented by the formulas (8) to (14), the salts thereof, and the solvates thereof is contained as the active ingredient.
[5] A protein folding method including: treating an unfolded protein in presence of at least one selected from the compounds represented by the formulas (1) to (14) defined in [1], the salts thereof, and the solvates thereof.
[6] A protein renaturation method including: treating an unfolded protein in presence of at least one selected from the compounds represented by the formulas (1) to (14) defined in [1], the salts thereof, and the solvates thereof.
[7] A protein solubilizing agent containing: as an active ingredient, at least one selected from the compounds represented by the formulas (1) to (14) defined in [1], the salts thereof, and the solvates thereof.

The present description includes the disclosure contents of Japanese patent applications No. 2021-033583 and No. 2022-020271 which are the basis for the priority of the present application.

### Advantageous Effects of Invention

With the protein folding agent according to the invention, a protein can be folded with high efficiency.

### Brief Description of Drawings

[FIG. 1A] FIG. 1A is a diagram illustrating a structure of a bovine pancreas trypsin inhibitor (BPTI) and a disulfide bond crosslinking position of each disulfide bond species in a folding pathway thereof.
[FIG. 1B] FIG. 1B is a graph illustrating a relation between HPLC retention time and each disulfide bond species when the disulfide bond crosslinking position in the BPTI using reversed-phase HPLC is determined.
[FIG. 2A] FIG. 2A is a graph illustrating a result of a reversed-phase HPLC analysis when a folding reaction of a reduced and denatured BPTI is performed using GSH/GSSG (Comparative Example 1).
[FIG. 2B] FIG. 2B is a graph illustrating a result of a reversed-phase HPLC analysis when a folding reaction of a reduced and denatured BPTI is performed using LDA-SH/LDA-SS (Example 1).
[FIG. 2C] FIG. 2C is a graph illustrating a result of a reversed-phase HPLC analysis when a folding reaction of a reduced and denatured BPTI is performed using BDA-SH/BDA-SS (Example 2).
[FIG. 3] FIG. 3 is a graph illustrating an activity recovery evaluation when a folding reaction of reduced and denatured RNase A is performed using LDA-SH/LDA-SS (Example 3), GSH/GSSG (Comparative Example 2), or GSSG.
[FIG. 4A] FIG. 4A is a graph illustrating a disulfide bond introduction reaction into reduced and denatured β2m using GSH/GSSG (Comparative Example 3) tracked by reversed-phase HPLC.
[FIG. 4B] FIG. 4B is a graph illustrating a disulfide bond introduction reaction into reduced and denatured β2m using LDA-SH/LDA-SS (Example 4) tracked by reversed-phase HPLC.
[FIG. 4C] FIG. 4C is a graph illustrating a disulfide bond introduction reaction into reduced and denatured β2m using BDA-SH/BDA-SS (Example 5) tracked by reversed-phase HPLC.
[FIG. 5] FIG. 5 is a graph illustrating quantification of oxidation type β2m due to disulfide bond introduction into reduced and denatured β2m by GSH/GSSG (Comparative Example 3), LDA-SH/LDA-SS (Example 4), or BDA-SH/BDA-SS (Example 5).
[FIG. 6] FIG. 6 is a graph illustrating an activity recovery evaluation when a folding reaction of reduced and denatured RNase A is performed using GSH/GSSG (Comparative Example 4), ImdM-SH/ImdM-SS (Example 6), or oPyM-SH/oPyM-SS (Example 7).
[FIG. 7] FIG. 7 is a graph illustrating an activity recovery evaluation when a folding reaction of reduced and denatured RNase A is performed using GSH/GSSG (Comparative Example 5), pPyM-SH-NMe·Cl/SS (Example 8), pPyM-SH-NMe·I/SS (Example 9), or oPyM-SH-NMe·I/SS (Example 10).
[FIG. 8] FIG. 8 is a photograph of each sample solution obtained in a β2m aggregation experiment by heating (before centrifugation).
[FIG. 9] FIG. 9 is a graph illustrating a residual rate of native type β2m in a supernatant liquid obtained by centrifuging each sample solution in the β2m aggregation experiment by heating.
[FIG. 10] FIG. 10 is a graph illustrating an activity recovery evaluation when a folding reaction of reduced and denatured RNase A is performed using GSH/GSSG (Comparative Example 4), ImdM-SH/ImdM-SS (Example 6), oPyM-SH/oPyM-SS (Example 7), or pPyM-SH/GSSG (Example 13).

### Description of Embodiments

Hereinafter, the invention will be described in detail based on embodiments.

The invention relates to a protein folding agent. The protein folding agent according to the invention contains, as an active ingredient, at least one selected from compounds represented by formulas (1) to (14), salts thereof, and solvates thereof. With the protein folding agent according to the invention, it is possible to introduce a disulfide bond into a protein with high efficiency, that is, at a high yield and/or at a high speed, as compared with a system using glutathione in the related art, and thus it is possible to progress oxidative folding of the protein with high efficiency. This leads to a reduction in time required for production of proteins used in preparations and the like and an improvement in yield amount, and thus an effect of large-scale production of protein preparations and a reduction in production cost can be obtained. Hereinafter, the compounds represented by the formulas (1) to (7), the salts thereof, and the solvates thereof are also simply referred to as "thiol compounds", and the compounds represented by the formulas (8) to (14), the salts thereof, and the solvates thereof are also simply referred to as "disulfide compounds".

The thiol compounds are compounds represented by the following formulas, the salts thereof, and the solvates thereof. 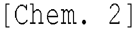 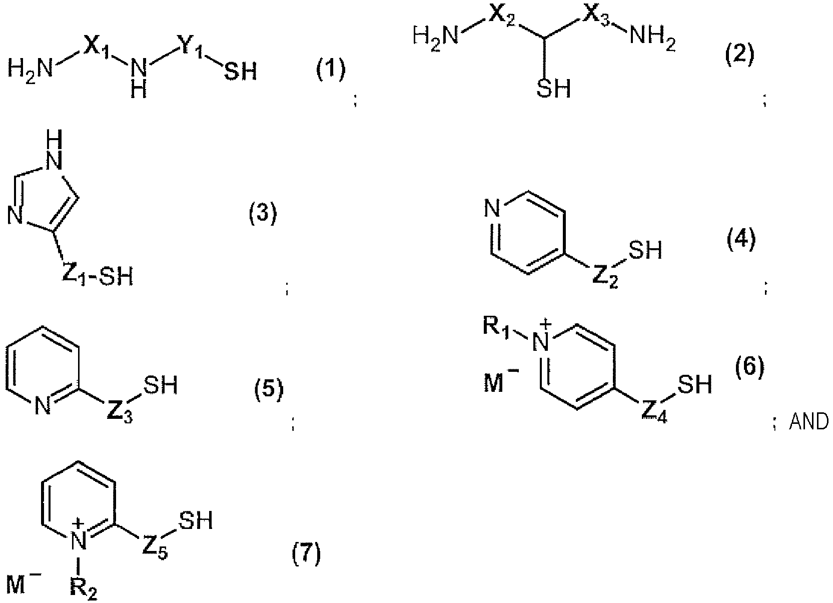 [In the formulas,
X₁, X₂, and X₃ are each independently an alkylene group having 1 to 10 carbon atoms which may contain 1 to 5 oxygen atoms in a molecular chain,
Y₁ is an alkylene group having 1 to 10 carbon atoms which may contain 1 to 5 oxygen atoms in a molecular chain,
Z₁, Z₂, Z₃, Z₄, and Z₅ are each independently an alkylene group having 1 to 10 carbon atoms which may contain 1 to 5 oxygen atoms in a molecular chain,
R₁ and R₂ are each independently an alkyl group having 1 to 24 carbon atoms, and
M⁻'s are each independently a chlorine ion, a bromine ion, or an iodide ion.]

In general, thiol compounds emit fetor, but all of the thiol compounds have suppressed fetor, which is an advantage over related compounds in the related art in terms of application. Further, the thiol compound is a molecule sufficiently smaller than a protein, and can thus be easily separated from the protein by size exclusion chromatography or dialysis after performing a disulfide bond introduction reaction.

The disulfide compounds are compounds represented by the following formulas, the salts thereof, and the solvates thereof. 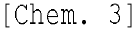 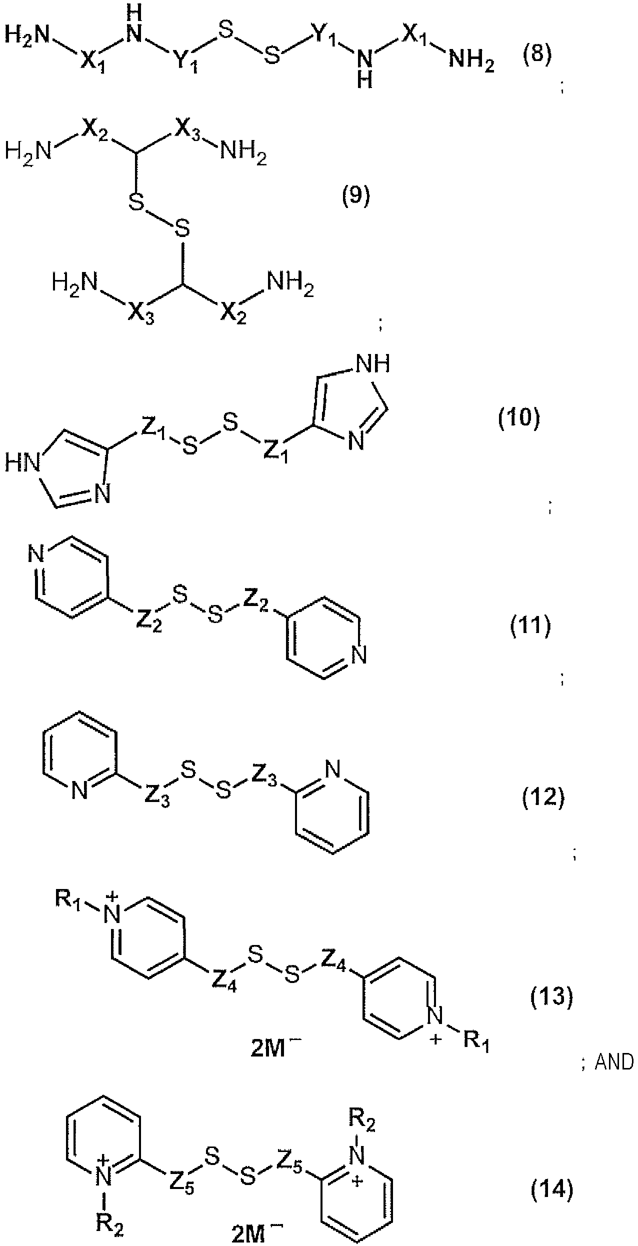 [In the formulas,
X₁, X₂, and X₃ are each independently an alkylene group having 1 to 10 carbon atoms which may contain 1 to 5 oxygen atoms in a molecular chain,
Y₁'s are each independently an alkylene group having 1 to 10 carbon atoms which may contain 1 to 5 oxygen atoms in a molecular chain,
Z₁, Z₂, Z₃, Z₄, and Z₅ are each independently an alkylene group having 1 to 10 carbon atoms which may contain 1 to 5 oxygen atoms in a molecular chain,
R₁ and R₂ are each independently an alkyl group having 1 to 24 carbon atoms, and
M⁻'s are each independently a chlorine ion, a bromine ion, or an iodide ion.]

Further, the disulfide compound is a molecule sufficiently smaller than a protein, and can thus be easily separated from the protein by size exclusion chromatography or dialysis after performing the disulfide bond introduction reaction.

A protein folding agent according to one embodiment of the invention contains, as an active ingredient, at least one selected from the compounds represented by the formulas (1) to (7), the salts thereof, and the solvates thereof, and at least one selected from the compounds represented by formulas (8) to (14), the salts thereof, and the solvates thereof. A molar ratio of the thiol compound to the disulfide compound contained in the protein folding agent according to the present embodiment is not particularly limited as long as folding efficiently proceeds, and is, for example, preferably 1:1 to 20:1, and more preferably 2:1 to 10:1. The protein folding agent according to the present embodiment may be sold or distributed in a state in which the thiol compound and the disulfide compound are mixed, or may be sold or distributed in a state in which the thiol compound and the disulfide compound are separately packaged as a kit, a set, or the like.

A protein folding agent according to another embodiment of the invention contains, as an active ingredient, at least one selected from the compounds represented by the formulas (1) to (7), the salts thereof, and the solvates thereof. The protein folding agent according to the present embodiment can be used in combination with at least one selected from the compounds represented by the formulas (8) to (14), the salts thereof, and the solvates thereof. In this case, a molar ratio of the thiol compound contained in the protein folding agent according to the present embodiment to the disulfide compound to be used in combination is not particularly limited as long as folding efficiently proceeds, and is, for example, preferably 1:1 to 20:1, and more preferably 2:1 to 10:1. The protein folding agent containing the thiol compound can be used in a protein folding reaction together with the separately obtained disulfide compound. Further, the disulfide compound to be used in combination may be one used in the related art, such as GSSG, and preferred embodiments in this case include, unless otherwise described, those cited in the above description for the disulfide compound.

A protein folding agent according to another embodiment of the invention contains, as an active ingredient, at least one selected from the compounds represented by the formulas (8) to (14), the salts thereof, and the solvates thereof. The protein folding agent according to the present embodiment can be used in combination with at least one selected from the compounds represented by the formulas (1) to (7), the salts thereof, and the solvates thereof. In this case, a molar ratio of the thiol compound to be used in combination to the disulfide compound contained in the protein folding agent according to the present embodiment is not particularly limited as long as folding efficiently proceeds, and is, for example, preferably 1:1 to 20:1, and more preferably 2:1 to 10:1. The protein folding agent containing the disulfide compound can be used in a protein folding reaction together with the separately obtained thiol compound. Further, the thiol compound to be used in combination may be one used in the related art such as GSH, and preferred embodiments in this case include, unless otherwise described, those cited in the above description for the thiol compound.

In the present description, the "active ingredient" refers to a substance that acts on a protein in a certain state in a process of folding the protein and a substance that acts on the substance, and has a function of promoting protein folding. In the present description, the "protein folding" includes (1) a step of unfolding a protein by reduction denaturation and (2) a step of performing oxidative folding on the unfolded protein. The protein folding agent according to the invention can fold a protein with high efficiency by introducing a disulfide bond into the protein with the thiol compound and the disulfide compound functioning as a reducing agent and an oxidizing agent, respectively, particularly in the oxidative folding reaction in the step (2).

When synthesizing the thiol compound and the disulfide compound, a general organic synthesis method for those skilled in the art can be appropriately employed. Specifically, reference can be made to a method for producing compounds corresponding to the thiol compounds represented by the formulas (1) to (7) and the disulfide compounds represented by the formulas (8) to (14) illustrated in Examples below.

Both the thiol compound and the disulfide compound may be in a form of a salt or a solvate. The salt is not particularly limited, and examples thereof include: salts with alkali metals such as sodium and potassium; salts with alkaline earth metals such as magnesium and calcium; ammonium salts; or salts with inorganic acids such as hydrochloric acid, phosphoric acid, nitric acid, sulfuric acid, and sulfurous acid; and salts with organic acids such as formic acid, acetic acid, propionic acid, butyric acid, oxalic acid, malonic acid, succinic acid, maleic acid, fumaric acid, tartaric acid, malic acid, mandelic acid, methanesulfonic acid, p-toluenesulfonic acid, and trifluoroacetic acid. Further, examples of the solvate include hydrates, and solvates with solvents such as alcohols (for example, methanol, ethanol, propanol, and isopropanol), acetone, tetrahydrofuran, dioxane, DMF, and DMSO.

Specific examples of the "alkylene group having 1 to 10 carbon atoms which may contain 1 to 5 oxygen atoms in a molecular chain" include, but are not limited to, a methylene group, an ethylene group, a propylene group, an isopropylene group, an n-butylene group, an isobutylene group, a tert-butylene group, an n-pentylene group, an isopentylene group, a neopentylene group, a hexylene group, a heptylene group, an octylene group, a nonylene group, a decylene group, and a polyoxyalkylene group (here, the alkylene is, for example, an ethylene group or a propylene group). Examples of the polyoxyalkylene group include, but are not limited to, *-(CH₂CH₂O)a- (here, a is an integer of 1 to 5, and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅), *-(OCH₂CH₂)b- (here, b is an integer of 1 to 5, and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅), *-(CH₂CH₂CH₂O)c- (here, c is an integer of 1 to 3, and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅), and *-(OCH₂CH₂CH₂)d- (here, d is an integer of 1 to 3, and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅).

Further, specific examples of the "alkylene group having 1 to 6 carbon atoms which may contain 1 to 3 oxygen atoms in a molecular chain" described in the present description include, but are not limited to, a methylene group, an ethylene group, a propylene group, an isopropylene group, an n-butylene group, an isobutylene group, a tert-butylene group, an n-pentylene group, an isopentylene group, a neopentylene group, a hexylene group, and a polyoxyalkylene group (here, the alkylene is, for example, an ethylene group or a propylene group). Examples of the polyoxyalkylene group include, but are not limited to, *-(CH₂CH₂O)a- (here, a is an integer of 1 to 3, and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅), *-(OCH₂CH₂)b- (here, b is an integer of 1 to 3, and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅), *-(CH₂CH₂CH₂O)c- (here, c is 1 or 2, and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅), and *-(OCH₂CH₂CH₂)d- (here, d is 1 or 2, and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅).

Further, specific examples of the "alkylene group having 1 to 4 carbon atoms which may contain 1 or 2 oxygen atoms in a molecular chain" described in the present description include, but are not limited to, a methylene group, an ethylene group, a propylene group, an isopropylene group, and a polyoxyalkylene group (here the alkylene is, for example, an ethylene group or a propylene group). Examples of the polyoxyalkylene group include, but are not limited to, *-(CH₂CH₂O)a- (here, a is an integer of 1 or 2), and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅), *-(OCH₂CH₂)b- (here, b is an integer of 1 or 2, and * represents a bonding site with - NH₂ for X₁, X₂, and X₃, a bonding site with -SH for Y₁, a bonding site with an imidazole ring for Z₁, and a bonding site with a pyridine ring for Z₂, Z₃, Z₄, and Z₅), *-(CH₂CH₂CH₂O)c- (here, c is 1, and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅), and *-(OCH₂CH₂CH₂)d- (here, d is 1, and * represents a bonding site with -NH₂ for X₁, X₂, and X₃, a bonding site with -SH for Y₁, a bonding site with an imidazole ring for Z₁, and a bonding site with a pyridine ring for Z₂, Z₃, Z₄, and Z₅).

Specific examples of the "alkyl group having 1 to 24 carbon atoms" include, but are not limited to, a methyl group, an ethyl group, an n-propyl group, an n-butyl group, an n-pentyl group, an n-hexyl group, an n-heptyl group, an n-octyl group, an n-nonyl group, n-decyl, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, an n-icosyl group, an n-heneicosyl group, an n-docosyl group, an n-tricosyl group, and an n-tetracosyl group.

A combination of the thiol compound and the disulfide compound used in the protein folding agent according to the invention is not particularly limited, and from a viewpoint of allowing the thiol compound and the disulfide compound to efficiently act as a reducing agent and an oxidizing agent, respectively, in shuffling of a disulfide bond in an oxidative folding reaction, it is preferable for them to be included in a combination having a correspondence relation (for example, a combination of the thiol compound represented by the formula (1) and the disulfide compound represented by the formula (8) which is a disulfide thereof), or to use them in combination. However, even in a case in which one of the thiol compound and the disulfide compound is used as an active ingredient of the protein folding agent according to the invention, in a folding step, the thiol compound and the disulfide compound can be made to exist in an equilibrium state in a folding liquid, and can be made to function as a reducing agent and an oxidizing agent in the shuffling of the disulfide bond.

In general, a reaction intermediate that is transiently generated in an oxidative folding process has a high aggregation property and is a factor reducing the yield. Accordingly, from a viewpoint of improving the yield of oxidative folding of a protein, the protein folding agent according to the invention preferably has a function of suppressing aggregation of the reaction intermediate. Incidentally, the thiol compound and the disulfide compound, which are active ingredients or ingredients to be used in combination in the protein folding agent according to the invention having such properties, can be used as active ingredients in a protein solubilizing agent.

Further, from a viewpoint of rapidly generating a non-native structure in addition to a native structure, the protein folding agent according to the invention preferably has a high activity of extremely rapidly introducing a disulfide bond. The protein exhibits completely different biochemical properties between the native structure and the non-native structure. In a case of a protein preparation, the native type protein preparation exhibits an expected drug efficacy, whereas the non-native type protein may produce a low drug efficacy and even a serious side effect. Therefore, from a viewpoint of examining the biochemical properties of the non-native structural protein and comprehensively clarifying potential side effects due to the drug efficacy of the non-native structural protein that may be contained as an impurity in the protein preparation (a misfolded protein), the protein folding agent according to the invention preferably has a function of providing a variety of non-native structural proteins at once. In the related art, there is no report on a compound capable of generating a variety of non-native structures at once for a protein having a plurality of cysteine pairs. As a method of preferentially providing a non-native structural protein, a method of replacing some cysteine residues in the protein with selenocysteine residues has been reported (N. Metanis et al., Angew. Chem. Int. Ed. 2012 51:5585-88). However, this method also converts the structure of the protein itself. Further, considering that only a few species of selenoproteins containing selenium in vivo have been reported, and physiological functions of these selenoproteins are still unknown and that excessive selenium intake is harmful to humans, it is considered that it is difficult to apply a selenium-containing low-molecular-weight compound in a pharmacological manner.

Further, the protein folding agent according to the invention preferably has a function of producing a non-native structural protein while suppressing aggregation. In general, a non-native structural protein has a high aggregation property and is difficult to be researched because a measurement system is remarkably limited. However, having the above functions is advantageous in examining biochemical properties and cytotoxicity of the non-native structural protein in a simple system, and is therefore considered to be useful for research such as a treatment method for a folding disease caused by the non-native structural protein. Incidentally, the thiol compound and the disulfide compound, which are active ingredients or ingredients to be used in combination in the protein folding agent according to the invention having such properties, can be used as active ingredients in a non-native structural protein solubilizing agent.

A protein to be folded using the protein folding agent according to the invention is not particularly limited as long as it contains at least one disulfide bond, and regardless of an origin, such as natural or artificial (a chemical synthesis method, a fermentation method, and a genetic recombination method), and a production method, a peptide, a polypeptide, a protein, and a complex thereof are included. The protein is not limited to a type, and includes, for example, an intracellular protein, an extracellular protein, a membrane protein, and a nuclear protein. Specific examples thereof include the following enzymes, recombinant proteins, and antibodies.

Examples of the enzyme include a hydrolase, an isomerase, an oxidoreductase, a transferase, a synthase, and a lyase. Examples of the hydrolase include protease, serine protease, amylase, lipase, cellulase, and glucoamylase. Examples of the isomerase include glucose isomerase. Examples of the oxidoreductase include protein disulfide isomerase and peroxidase. Examples of the transferase include acyltransferase and sulfotransferase. Examples of the synthase include fatty acid synthase, phosphate synthase, and citrate synthase. Examples of the lyase include pectin lyase.

Examples of the recombinant protein include a protein preparation and a vaccine. Since a recombinant protein produced by genetic engineering using a prokaryote such as E. coli, a eukaryote such as a yeast, or a heterogenous expression system such as a cell-free extraction system is often obtained as an insoluble and inactive aggregate, a so-called inclusion body, the protein folding agent according to the invention can be suitably used. Examples of the protein preparation include interferon a, interferon β, interleukins 1 to 12, growth hormones, erythropoietin, insulin, a granular colony stimulation factor (G-CSF), a tissue plasminogen activator (TPA), defensins, natriuretic peptides, a blood coagulation factor 2, somatomedin, glucagon, a growth hormone-releasing factor, serum albumin, and calcitonin. Examples of the vaccine include a hepatitis A vaccine, a hepatitis B vaccine, and a hepatitis C vaccine. The antibody is not particularly limited, and examples thereof include a medical antibody.

When the protein folding agent according to the invention is used for oxidative folding, a step of treating an unfolded protein in presence of the protein folding agent according to the invention (hereinafter, also simply referred to as a treatment step) is performed by, for example, blending an unfolded protein, the protein folding agent according to the invention, and a thiol compound or disulfide compound to be used in combination in some cases in a folding buffer and mixing them by stirring or the like.

The protein unfolding can be performed by appropriately adopting a general method for those skilled in the art. For example, guanidine hydrochloride, urea, thiourea, or a combination thereof can be used to obtain an unfolded protein. In addition, the protein unfolding can be performed by a method described in "8) Preparation of Reduced and Denatured Protein" in Examples below.

Treatment time in the treatment step can be appropriately adjusted such that a target protein can be obtained with high efficiency. Further, a temperature can be appropriately selected depending on heat resistance of a target protein, and is, for example, in a range of 0°C to 100°C, preferably in a range of 4°C to 40°C.

A concentration (a total amount) of the thiol compound and the disulfide compound as the oxidizing agent used in the treatment step is not particularly limited, and is, for example, usually 0.01 mM to 100 mM with respect to a solution containing the target protein (for example, the folding buffer). Further, a concentration of the unfolded protein contained in the solution containing the target protein (for example, the folding buffer) is usually 0.01 µM to 100 µM.

In the solution containing the target protein (for example, the folding buffer) in the treatment step, a molar ratio of the thiol compound as the reducing agent (a total amount) to the disulfide compound as the oxidizing agent (a total amount) is not particularly limited as long as introduction of the disulfide bond for obtaining the target protein efficiently proceeds, and is, for example, 1:1 to 20:1.

The folding buffer is not particularly limited as long as it does not have a concentration and composition that lose a function of the target protein, and specific examples thereof include an amine buffer such as a Tris buffer, a MES buffer, and a trisin buffer, a phosphate buffer, and various Good's buffers. A pH of the folding buffer can be adjusted in a range of usually pH 4 to 10, preferably pH 5 to 9, and more preferably pH 7 to 9.

Various additives can be added to the folding buffer in addition to the protein folding agent according to the invention and, as necessary, the thiol compound or the disulfide compound to be used in combination. Examples of such additives include: salts such as sodium chloride and calcium chloride; buffers such as a citrate, a phosphate, and an acetate; bases such as sodium hydroxide; acids such as hydrochloric acid and acetic acid; and organic solvents such as methanol, ethanol, and propanol. Further, in addition to the folding agent according to the invention and the additives, a surfactant, a pH adjuster, or a protein stabilizer can be blended into the buffer agent. Those skilled in the art can appropriately adjust an amount of use thereof.

The thiol compound represented by the formulas (1) to (7) and the disulfide compound represented by the formulas (8) to (14), which are active ingredients or ingredients to be used in combination in the protein folding agent according to the invention, will be described below.

The following thiol compound (1) and the following disulfide compound (8) as active ingredients in the protein folding agent according to the invention:, and a combination thereof as an ingredient to be used in combination are preferable from a viewpoint of obtaining a native structural protein at a high yield by oxidative folding.

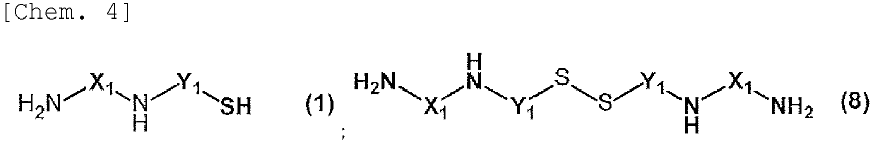

[In the formulas,
X₁'s are each independently an alkylene group having 1 to 10 carbon atoms which may contain 1 to 5 oxygen atoms in a molecular chain, and
Y₁'s are each independently an alkylene group having 1 to 10 carbon atoms which may contain 1 to 5 oxygen atoms in a molecular chain.]

A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect, a protein having a plurality of disulfide bonds, for example, a protein having four disulfide bonds such as RNase A is preferable.

Further, the compounds or combinations thereof are preferable from a viewpoint of suppressing aggregation of reaction intermediates in oxidative folding and obtaining a native structural protein at a high yield. A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect, a protein having a plurality of disulfide bonds, for example, a protein having three disulfide bonds such as a bovine pancreas trypsin inhibitor is preferable.

Further, the compounds or combinations thereof are preferable from a viewpoint of obtaining a native structural protein at a high yield and a high speed by oxidative folding. A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect, a protein having one disulfide bond such as β₂-microglobulin (β2m) is preferable.

From the viewpoint of obtaining the effect as described above, X₁ is preferably an alkylene group having 1 to 6 carbon atoms which may contain 1 to 3 oxygen atoms in the molecular chain, and more preferably an alkylene group having 1 to 4 carbon atoms which may contain 1 or 2 oxygen atoms in the molecular chain. Further, from the viewpoint of obtaining the effect as described above, Y₁ is preferably an alkylene group having 1 to 6 carbon atoms which may contain 1 to 3 oxygen atoms in the molecular chain, and more preferably an alkylene group having 1 to 4 carbon atoms which may contain 1 or 2 oxygen atoms in the molecular chain.

The following thiol compound (2) and the following disulfide compound (9) as active ingredients in the protein folding agent according to the invention, and a combination thereof as an ingredient to be used in combination are preferable from a viewpoint of extremely rapidly introducing a disulfide bond in oxidative folding and generating various non-native structures at once.

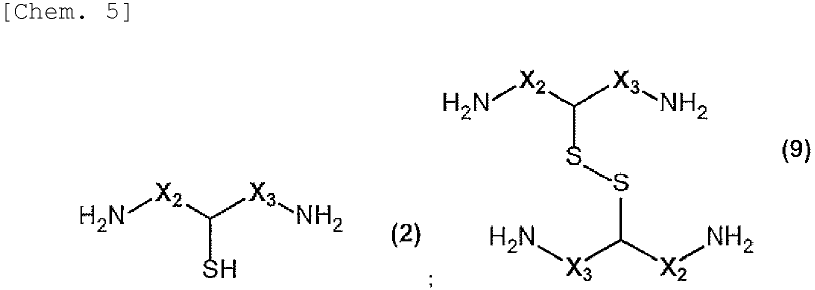

[In the formulas,
X₂ and X₃ are each independently an alkylene group having 1 to 10 carbon atoms which may contain 1 to 5 oxygen atoms in a molecular chain.]

A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect, a protein having a plurality of disulfide bonds, for example, a protein having three disulfide bonds such as a bovine pancreas trypsin inhibitor is preferable.

Further, the compounds or combinations thereof are preferable from a viewpoint of generating a non-native structural protein while suppressing aggregation in oxidative folding. A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect, a protein having a plurality of disulfide bonds, for example, a protein having three disulfide bonds such as a bovine pancreas trypsin inhibitor is preferable.

Further, the compounds or combinations thereof are preferable from a viewpoint of obtaining a native structural protein at a high yield and a high speed by oxidative folding. A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect, a protein having one disulfide bond such as β₂-microglobulin (β2m) is preferable.

From the viewpoint of obtaining the effect as described above, X₂ is preferably an alkylene group having 1 to 6 carbon atoms which may contain 1 to 3 oxygen atoms in the molecular chain, and more preferably an alkylene group having 1 to 4 carbon atoms which may contain 1 or 2 oxygen atoms in the molecular chain. Further, from the viewpoint of obtaining the effect as described above, X₃ is preferably an alkylene group having 1 to 6 carbon atoms which may contain 1 to 3 oxygen atoms in the molecular chain, and more preferably an alkylene group having 1 to 4 carbon atoms which may contain 1 or 2 oxygen atoms in the molecular chain.

The following thiol compound (3) and the following disulfide compound (10) as active ingredients in the protein folding agent according to the invention, and a combination thereof as an ingredient to be used in combination are preferable from a viewpoint of obtaining a native structural protein at a high yield by oxidative folding.

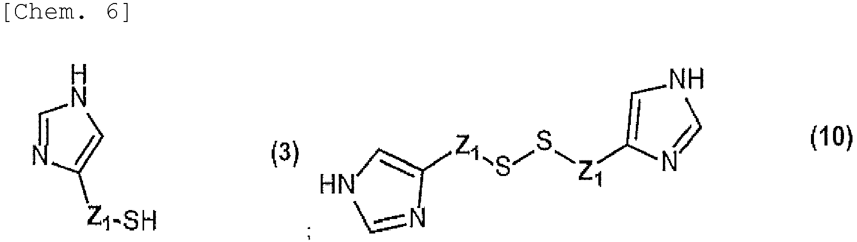

[In the formulas,
Z₁'s are each independently an alkylene group having 1 to 10 carbon atoms which may contain 1 to 5 oxygen atoms in a molecular chain.]

A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect, a protein having a plurality of disulfide bonds, for example, a protein having four disulfide bonds such as RNase A is preferable.

Further, from the viewpoint of obtaining the effect as described above, Z₁ is preferably an alkylene group having 1 to 6 carbon atoms which may contain 1 to 3 oxygen atoms in the molecular chain, and more preferably an alkylene group having 1 to 4 carbon atoms which may contain 1 or 2 oxygen atoms in the molecular chain.

The following thiol compound (4) and the following disulfide compound (11) as active ingredients in the protein folding agent according to the invention, and a combination thereof as an ingredient to be used in combination are preferable from a viewpoint of promoting oxidative folding.

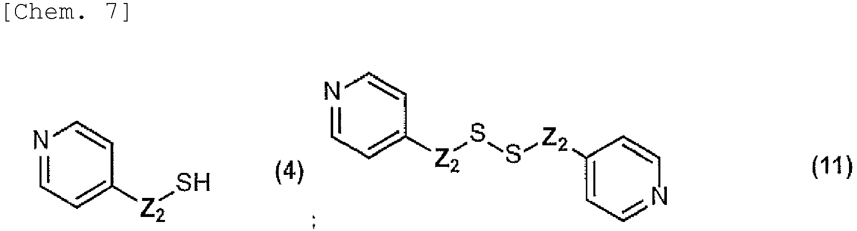

[In the formulas,
Z₂'s are each independently an alkylene group having 1 to 10 carbon atoms which may contain 1 to 5 oxygen atoms in a molecular chain.]

A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect as described above, Z₂ is preferably an alkylene group having 1 to 6 carbon atoms which may contain 1 to 3 oxygen atoms in the molecular chain, and more preferably an alkylene group having 1 to 4 carbon atoms which may contain 1 or 2 oxygen atoms in the molecular chain.

The following thiol compound (5) and the following disulfide compound (12) as active ingredients in the protein folding agent according to the invention, and a combination thereof as an ingredient to be used in combination are preferable from a viewpoint of obtaining a native structural protein by oxidative folding in a short time and at a high yield.

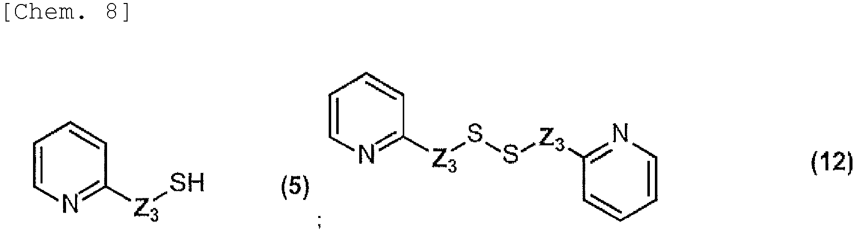

[In the formulas,
Z₃'s are each independently an alkylene group having 1 to 10 carbon atoms which may contain 1 to 5 oxygen atoms in a molecular chain.]

A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect, a protein having a plurality of disulfide bonds, for example, a protein having four disulfide bonds such as RNase A is preferable.

From a viewpoint of obtaining the effect as described above, Z₃ is preferably an alkylene group having 1 to 6 carbon atoms which may contain 1 to 3 oxygen atoms in the molecular chain, and more preferably an alkylene group having 1 to 4 carbon atoms which may contain 1 or 2 oxygen atoms in the molecular chain.

The following thiol compound (6) and the following disulfide compound (13) as active ingredients in the protein folding agent according to the invention, and a combination thereof as an ingredient to be used in combination are preferable from a viewpoint of obtaining a native structural protein at a high yield by oxidative folding of the protein.

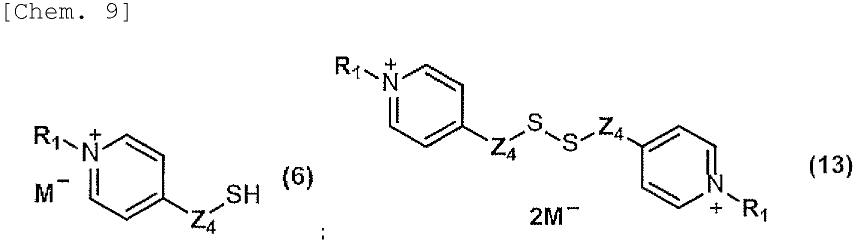

[In the formulas,
Z₄'s are each independently an alkylene group having 1 to 10 carbon atoms which may contain 1 to 5 oxygen atoms in a molecular chain,
R₁'s are each independently an alkyl group having 1 to 24 carbon atoms, and
M⁻'s are each independently a chlorine ion, a bromine ion, or an iodide ion.]

A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect, a protein having a plurality of disulfide bonds, for example, a protein having four disulfide bonds such as RNase A is preferable.

From a viewpoint of obtaining the effect as described above, Z₄ is preferably an alkylene group having 1 to 6 carbon atoms which may contain 1 to 3 oxygen atoms in the molecular chain, and more preferably an alkylene group having 1 to 4 carbon atoms which may contain 1 or 2 oxygen atoms in the molecular chain. Further, from the viewpoint of obtaining the effect as described above, R₁ is preferably an alkyl group having 1 to 6 carbon atoms, and more preferably an alkyl group having 1 or 2 carbon atoms. Further, from the viewpoint of obtaining the effect as described above, M⁻ is preferably a chlorine ion or an iodide ion.

Further, the thiol compound (6), the disulfide compound (13), and a combination thereof aggregate in water due to hydrophobic interaction of the R₁ group to form a micelle, and an amphipathic effect of a hydrophobic internal space and a hydrophilic surface of the micelle suppresses aggregation of denatured proteins and protein folding intermediates, and thus oxidative folding can be promoted by introducing a disulfide bond into a protein with high efficiency. In this case, R₁ is preferably an alkyl group having 3 to 18 carbon atoms, and more preferably an alkyl group having 4 to 12 carbon atoms. A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin.

The following thiol compound (7) and the following disulfide compound (14) as active ingredients in the protein folding agent according to the invention, and a combination thereof as an ingredient to be used in combination are preferable from a viewpoint of obtaining a native structural protein at a high yield by oxidative folding.

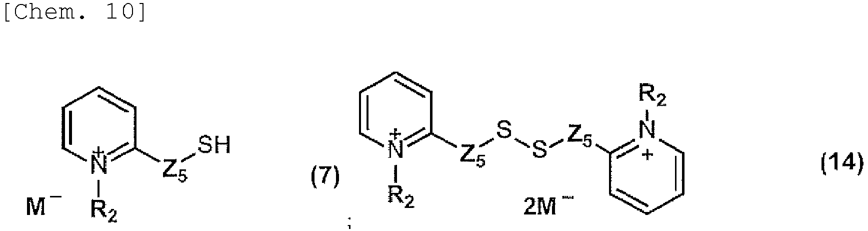

[In the formulas,
Z₅'s are each independently an alkylene group having 1 to 10 carbon atoms which may contain 1 to 5 oxygen atoms in a molecular chain,
R₂'s are each independently an alkyl group having 1 to 24 carbon atoms, and
M⁻'s are each independently a chlorine ion, a bromine ion, or an iodide ion.]

A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin. From a viewpoint of obtaining the effect, a protein having a plurality of disulfide bonds, for example, a protein having four disulfide bonds such as RNase A is included.

From a viewpoint of obtaining the effect as described above, Z₅ is preferably an alkylene group having 1 to 6 carbon atoms which may contain 1 to 3 oxygen atoms in the molecular chain, and more preferably an alkylene group having 1 to 4 carbon atoms which may contain 1 or 2 oxygen atoms in the molecular chain. Further, from the viewpoint of obtaining the effect as described above, R₂ is preferably an alkyl group having 1 to 6 carbon atoms, and more preferably an alkyl group having 1 or 2 carbon atoms. Further, from the viewpoint of obtaining the effects as described above, M⁻ is preferably an iodide ion.

Further, the thiol compound (7), the disulfide compound (14), and a combination thereof aggregate in water due to hydrophobic interaction of the R₂ group to form a micelle, and an amphipathic effect of a hydrophobic internal space and a hydrophilic surface of the micelle suppresses aggregation of denatured proteins and protein folding intermediates, and thus oxidative folding can be promoted by introducing a disulfide bond into a protein with high efficiency. In this case, R₂ is preferably an alkyl group having 3 to 18 carbon atoms, and more preferably an alkyl group having 4 to 12 carbon atoms. A protein to be folded in this case is not particularly limited, and examples thereof include the protein described above, and further include RNase A, a bovine pancreas trypsin inhibitor, β₂-microglobulin, insulin, a major histocompatibility complex, and immunoglobulin.

The invention also relates to a protein folding method including a step of treating an unfolded protein in presence of at least one selected from the compounds represented by the formulas (1) to (14), the salts thereof, and the solvates thereof. Examples of the step of treating an unfolding protein in the presence of the compound in the folding method according to the invention (hereinafter, also simply referred to as a treatment step) include a step of bringing the unfolded protein into contact with the compound, and specifically include a step of blending the unfolded protein and the compound in a folding buffer and mixing them by stirring or the like, and after this step, further include, as necessary, a step of leaving the folded protein for a certain period of time in order to more sufficiently advance folding. Preferred embodiments of the folding method according to the invention include, unless otherwise described, those cited in the above description for the folding agent according to the invention.

The invention also relates to a protein renaturation method including a step of treating and folding an unfolded protein in presence of at least one selected from the compounds represented by the formulas (1) to (14), the salts thereof, and the solvates thereof. The protein renaturation method according to the invention also includes a step of treating and folding an unfolded protein in the presence of the compound (hereinafter, also simply referred to as a treatment step), and after this step, further includes, as necessary, a step of isolating the folded protein (hereinafter, also referred to as an isolation step). Preferred embodiments of the protein renaturation method according to the invention include, unless otherwise described, those cited in the above descriptions for the folding agent according to the invention and the folding method according to the invention.

Examples of the isolation step include isolating a target normal protein (folded protein) from a protein suspension liquid obtained in the folding step, using column chromatography or the like. Examples of a filler used for the column chromatography include silica, dextran, agarose, cellulose, acrylamide, and a vinyl polymer. Commercially available products include Sephadex series, Sephacryl series, Sepharose series (all of them are from Pharmacia Corporation), Bio-Gel series (Bio-Rad Corporation). Further, the target normal protein (folded protein) may be isolated by dialysis.

Further, the invention also relates to a protein solubilizing agent containing, as an active ingredient, at least one selected from the compounds represented by the formulas (1) to (14), the salts thereof, and the solvates thereof. The solubilizing agent according to the invention can suppress and solubilize aggregation of the native structural or non-native structural proteins. As described above with respect to the protein folding agent according to the invention, the thiol compounds represented by the formulas (1) to (7) and the disulfide compounds represented by the formulas (8) to (14), which are active ingredients or ingredients to be used in combination in the protein folding agent according to the invention, have a function of suppressing aggregation of native structural or non-native structural proteins, in particular, a function of suppressing aggregation of reaction intermediates and native structural proteins generated in protein oxidative folding, and a function of suppressing aggregation of non-native structural proteins in an oxidative folding reaction for generating one or more types of non-native structural proteins, and thus function as a solubilizing agent. Preferred embodiments of the compounds represented by the formulas (1) to (14), the target protein, and the like include those cited in the above description for the protein folding agent according to the invention.

### Examples

Hereinafter, the invention will be described more specifically with reference to examples. However, the technical scope of the invention is not limited to these examples.

### 1) Syntheses of LDA-SH (2-((2-aminoethyl)amino)ethane-1-thiol) and LDA-SS

The compounds (corresponding to the compounds represented by the formulas (1) and (8), respectively) were synthesized by the following scheme.

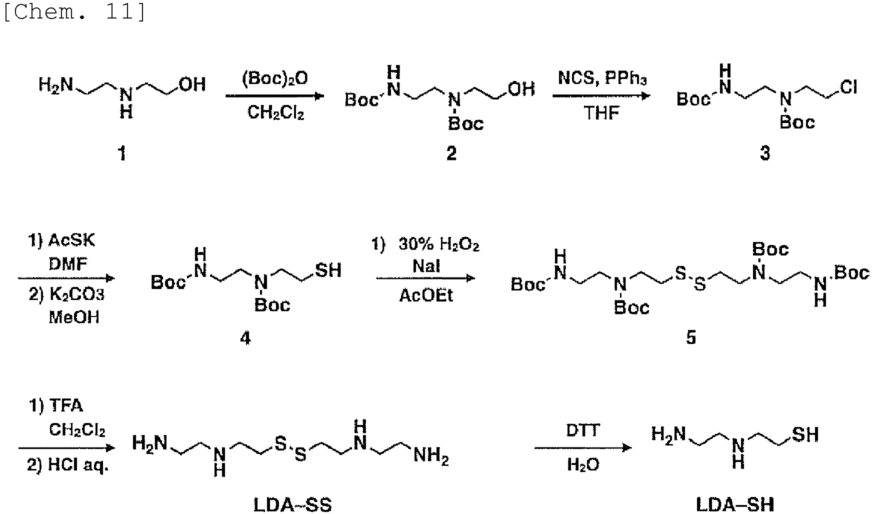

### 1-1) Synthesis of Compound 2

Compound 1 (1.90 g, 18.3 mmol, Tokyo Chemical Industry) was dissolved in dehydrated methylene chloride (25 mL, Kanto Chemical) on an ice bath under a nitrogen atmosphere. To this was added (Boc)₂O (5.22 g, 23.9 mmol, Kanto Chemical), followed by heating to room temperature. After 19 hours, the solvent was distilled off under reduced pressure, and water (25 mL) was added, followed by extraction with ethyl acetate (25 mL, twice, Kishida Chemical). The ethyl acetate solution after extraction was washed with a saturated saline solution (25 mL). To the recovered ethyl acetate solution was added sodium sulfate (Kishida Chemical), followed by filtration. The filtrate was distilled off under reduced pressure, followed by silica gel column chromatography (Kanto Chemical), to give Compound 2. Yield amount: 3.62 g, yield: 65%.

### 1-2) Synthesis of Compound 3

N-chlorosuccinimide (NCS, 0.849 g, 6.36 mmol, Kishida Chemical) was dissolved in dehydrated tetrahydrofuran (THF, 36 mL, Kanto Chemical) at room temperature under a nitrogen atmosphere. To this was added dropwise a dehydrated THF solution (13 mL) of PPh₃ (1.67 g, 6.38 mmol, FUJIFILM Wako Pure Chemical). After 20 minutes, a dehydrated THF solution (13 mL) of Compound 2 (1.75 g, 5.74 mmol) was added dropwise. After 11 hours, the solvent was distilled off under reduced pressure. The residue was subjected to silica gel column chromatography (Kanto Chemical), to give Compound 3. Yield amount: 0.60 g, yield: 32%.

### 1-3) Synthesis of Compound 4

Compound 3 (0.597 g, 1.85 mmol) was dissolved in dehydrated N,N-dimethylformamide (DMF, 3 mL, Kanto Chemical) at room temperature under a nitrogen atmosphere. To this was added potassium thioacetate (AcSK, 0.285 g, 2.50 mmol, Tokyo Chemical Industry), followed by heating to 90°C. After 12 hours, water (20 mL) was added, followed by extraction with ethyl acetate (25 mL, twice, Kishida Chemical). The ethyl acetate solution after extraction was washed with a saturated saline solution (25 mL). To the recovered ethyl acetate solution was added sodium sulfate (Kishida Chemical), followed by filtration. The filtrate was distilled off under reduced pressure. To the obtained residue was added dehydrated methanol (5 mL, Kanto Chemical) at room temperature under a nitrogen atmosphere, followed by adding sodium carbonate (0.757 g, 5.48 mmol, Kishida Chemical). After 1 hour, hydrochloric acid (1 M, Kishida Chemical) was added until the pH reached 9 from 8. To the reaction mixture was added water (25 mL), followed by extraction with methylene chloride (25 mL, 3 times, AGC). The methylene chloride solution after extraction was washed with a saturated saline solution (25 mL, twice). To the recovered methylene chloride solution was added sodium sulfate (Kishida Chemical), followed by filtration. The filtrate was distilled off under reduced pressure, and the residue was subjected to silica gel column chromatography (Kanto Chemical), to give Compound 4. Yield amount: 0.257 g, yield: 43%.

### 1-4) Synthesis of Compound 5

Compound 4 (0.257 g, 0.803 mmol) was dissolved in ethyl acetate (5.0 mL, Kanto Chemical) at room temperature in air. To this was added sodium iodide (6.9 mg, 46 µmol, Kishida Chemical), followed by adding a 30% hydrogen peroxide solution (30 mL, Kishida Chemical). After stirring for 1 hour, water (20 mL) was added, followed by extraction with ethyl acetate (50 mL, twice, Kishida Chemical). The ethyl acetate solution after extraction was washed with a saturated saline solution (25 mL). To the recovered ethyl acetate solution was added sodium sulfate (Kishida Chemical), followed by filtration. The filtrate was distilled off under reduced pressure, to give Compound 5. Yield amount: 0.241 g, yield: 94%.

### 1-5) Synthesis of LDA-SS

Compound 5 (256 mg, 0.401 mmol) was dissolved in methylene chloride (3 mL, AGC) at room temperature in air. To this was added trifluoroacetic acid (2 mL, Kishida Chemical). After stirring for 1 hour, the solvent was distilled off under reduced pressure. To the residue was added methylene chloride (10 mL, AGC), followed by extraction with water (10 mL). The aqueous solution after extraction was distilled off under reduced pressure, followed by high performance liquid chromatography (apparatus: PU-4086 and UV-4075 manufactured by JASCO, column: TA12S05-2520WX), to give LDA-SS. Yield amount: 87 mg, yield: 60%.

### 1-6) Synthesis of LDA-SH

LDA-SS (76 mg, 0.632 mmol) was dissolved in water (2 mL) at room temperature under a nitrogen atmosphere. To this was added dithiothreitol (112 mg, 0.726 mmol, NACALAI TESQUE). After stirring for 24 hours, high performance liquid chromatography (apparatus: PU-4086 and UV-4075 manufactured by JASCO, column: TA12S05-2520WX), to give LDA-SH. Yield amount: 33 mg, yield: 43%.

### 2) Syntheses of BDA-SH (1,3-diaminopropane-2-thiol) and BDA-SS

The compounds (corresponding to the compounds represented by the formulas (2) and (9), respectively) were synthesized by the following scheme.

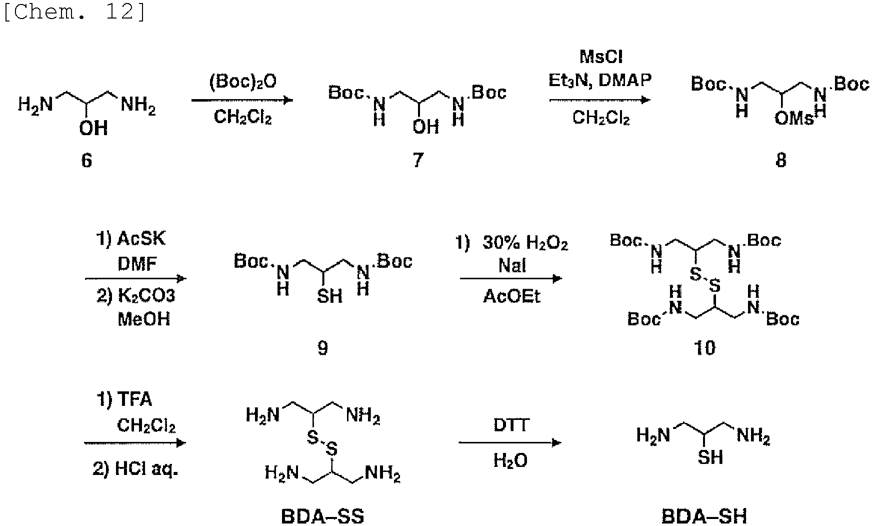

### 2-1) Synthesis of Compound 7

Compound 6 (0.539 g, 5.98 mmol) was dissolved in dehydrated methanol (30 mL, Kanto Chemical) at room temperature under a nitrogen atmosphere. After heating to 45°C, (Boc)₂O (2.84 g, 13.0 mmol, Kanto Chemical) and triethylamine (6.91 mL, SIGMA) were added. After 20 hours, the solvent was distilled off under reduced pressure, and the obtained residue was subjected to silica gel column chromatography (Kanto Chemical), to give Compound 7. Yield amount: 1.72 g, yield: 99%.

### 2-2) Synthesis of Compound 8

Compound 7 (1.57 g, 5.41 mmol) was dissolved in dehydrated methylene chloride (57 mL, Kanto Chemical) at room temperature under a nitrogen atmosphere. After cooling to 0°C, triethylamine (2.20 mL, SIGMA) and mesyl chloride (0.544 mL, 7.03 mmol, Tokyo Chemical Industry) were added. After stirring for 5 hours, water (30 mL) was added, followed by extraction with methylene chloride (30 mL, twice, AGC). The recovered methylene chloride solution was washed with a saturated sodium bicarbonate solution (10 mL) and a saturated saline solution (10 mL), followed by adding sodium sulfate (Kishida Chemical) and filtration. The filtrate was distilled off under reduced pressure, and the residue was subjected to silica gel column chromatography (Kanto Chemical), to give Compound 8. Yield amount: 1.64 g, yield: 82%.

### 2-3) Synthesis of Compound 9

Compound 8 (0.756 g, 2.05 mmol) was dissolved in dehydrated N,N-dimethylformamide (DMF, 13 mL, Kanto Chemical) at room temperature under a nitrogen atmosphere. Potassium thioacetate (0.392 g, 3.428 mmol, Tokyo Chemical Industry) and triethylamine (0.30 mL, SIGMA) were added. After stirring at 60°C for 13 hours, water (50 mL) was added, followed by extraction with ethyl acetate (50 mL, 3 times, Kishida Chemical). The recovered ethyl acetate solution was washed with water (50 mL, twice) and a saturated saline solution (10 mL), followed by adding sodium sulfate (Kishida Chemical) and filtration. The filtrate was distilled off under reduced pressure, and the residue was subjected to silica gel column chromatography (Kanto Chemical), to give a thioacetyl intermediate. The thioacetyl intermediate (0.346 g, 0.994 mmol) was dissolved in dehydrated methanol (3 mL, Kanto Chemical) at room temperature under a nitrogen atmosphere, followed by adding potassium carbonate (0.414 g, 2.998 mmol, Kishida Chemical). After stirring for 2 hours, the solvent was distilled off under reduced pressure. Water (10 mL) was added, followed by extraction with methylene chloride (10 mL, 3 times, AGC). The recovered methylene chloride solution was washed with a saturated ammonium chloride aqueous solution (10 mL, Kishida Chemical) and a saturated saline solution (10 mL), followed by adding sodium sulfate (Kishida Chemical) and filtration. The filtrate was distilled off under reduced pressure, and the residue was subjected to silica gel column chromatography (Kanto Chemical), to give Compound 9. Yield amount: 0.275 g, yield: 43%.

### 2-4) Synthesis of Compound 10

Compound 9 (0.279 g, 0.897 mmol) was dissolved in ethyl acetate (5 mL, Kanto Chemical) at room temperature, followed by adding sodium iodide (11.8 mg, 0.079 mmol, Kishida Chemical) and a 30% hydrogen peroxide solution (0.05 mL, Kishida Chemical). After stirring for 1 hour, water (30 mL) was added, followed by extraction with ethyl acetate (30 mL, twice). The recovered ethyl acetate solution was washed with a saturated saline solution (10 mL), followed by adding sodium sulfate and filtration. The filtrate was distilled off under reduced pressure, and the residue was subjected to silica gel column chromatography (Kanto Chemical), to give Compound 10. Yield amount: 0.274 g, yield: 99%.

### 2-5) Synthesis of BDA-SS

Compound 10 (0.274 g, 0.449 mmol) was dissolved in methylene chloride (5 mL, AGC), followed by cooling to 0°C. Trifluoroacetic acid (1 mL, Kishida Chemical) was added, followed by heating to room temperature. After stirring for 2 hours, the solvent was distilled off under reduced pressure. To the obtained residue was added chloroform (10 mL, Kishida Chemical), followed by extraction with water (10 mL). The solvent in the obtained aqueous solution was distilled off under reduced pressure, and to the residue was added hydrochloric acid (1 M, 5 mL, Kishida Chemical). After stirring for 1 hour, the solvent was distilled off under reduced pressure to give BDA-SS. Yield amount: 0.103 g, yield: 64%.

### 2-6) Synthesis of BDA-SH

BDA-SS (92.3 mg, 0.259 mmol) was dissolved in water (3 mL) at room temperature under a nitrogen atmosphere, followed by adding dithiothreitol (183 mg, 1.19 mmol, NACALAI TESQUE). After stirring for 24 hours, a mixed solvent of chloroform (Kishida Chemical) and 1-propanol (Kishida Chemical) (1:5, 5 mL) was added, followed by extraction with water (5 mL). The solvent in the obtained aqueous solution was distilled off under reduced pressure, to give BDA-SH. Yield amount: 85.5 mg, yield: 93%.

### 3) Syntheses of ImdM-SH ((1H-imidazol-4-yl)methanethiol) and ImdM-SS

The compounds (corresponding to the compounds represented by the formulas (3) and (10), respectively) were synthesized by the following scheme.

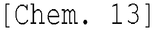

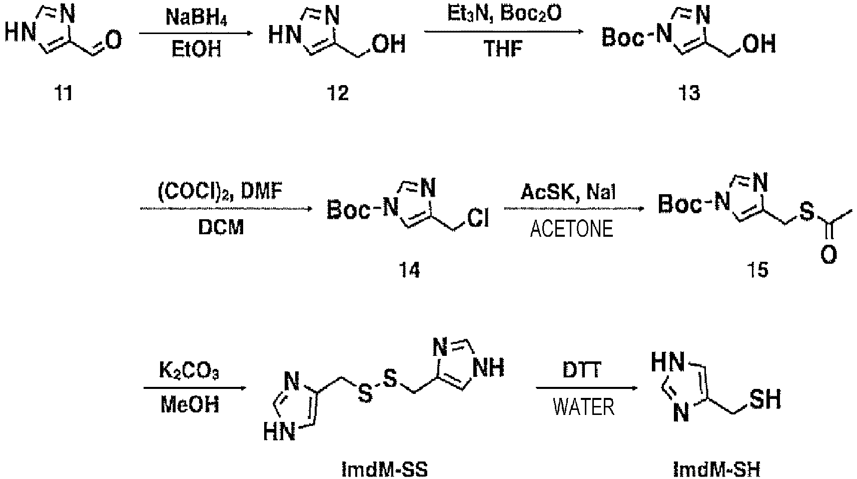

### 3-1) Synthesis of Compound 12

Compound 11 (5.512 g, 57.36 mmol, Tokyo Chemical Industry) was dissolved in dehydrated ethanol (100 mL, Kanto Chemical) at room temperature under a nitrogen atmosphere. After cooling to 0°C, sodium borohydride (2.165 g, 57.24 mmol, Tokyo Chemical Industry) was added. After stirring for 3 hours, water (10 mL) was added, and the solvent was distilled off under reduced pressure. The obtained residue was subjected to silica gel column chromatography (Kanto Chemical), to give Compound 12. Yield amount: 4.10 g, yield: 73%.

### 3-2) Synthesis of Compound 13

Compound 12 (0.565 g, 5.76 mmol) was dissolved in dehydrated tetrahydrofuran (1.7 mL, Kanto Chemical Industry) and triethylamine (0.85 mL, SIGMA) at room temperature under a nitrogen atmosphere. A dehydrated tetrahydrofuran solution (8.1 mL, Kanto Chemical) of (Boc)₂O (1.37 g, 6.28 mmol, Kanto Chemical) was added dropwise. After stirring for 13 hours, the solvent was distilled off under reduced pressure, and water (15 mL) was added, followed by extraction with methylene chloride (20 mL, 5 times, Kishida Chemical). To the recovered methylene chloride solution was added sodium sulfate (Kishida Chemical), followed by filtration. The filtrate was distilled off under reduced pressure, and the residue was subjected to silica gel column chromatography (Kanto Chemical), to give Compound 13. Yield amount: 0.763 g, yield: 67%.

### 3-3) Synthesis of Compound 14

Compound 13 (0.917 g, 4.63 mmol) was dissolved in dehydrated methylene chloride (8.4 mL, Kanto Chemical) and dehydrated N,N-dimethylformamide (DMF, 0.2 mL, Kanto Chemical) at room temperature under a nitrogen atmosphere. After cooling to 0°C, a dehydrated methylene chloride solution (7.0 mL, Kanto Chemical) of oxalyl chloride (1.22 g, 9.58 mmol, Kishida Chemical) was added dropwise. After heating to room temperature and stirring for 1.5 hours, water (15 mL) was added, followed by extraction with methylene chloride (15 mL, twice, Kishida Chemical). To the recovered methylene chloride solution was added sodium sulfate (Kishida Chemical), followed by filtration. The filtrate was distilled off under reduced pressure, and the residue was subjected to silica gel column chromatography (Kanto Chemical), to give Compound 14. Yield amount: 0.432 g, yield: 43%.

### 3-4) Synthesis of Compound 15

Compound 14 (0.168 g, 0.774 mmol) was dissolved in dehydrated acetone (4.7 mL, Kanto Chemical) at room temperature under a nitrogen atmosphere. Sodium iodide (0.233 g, 1.55 mmol, Kishida Chemical) and potassium thioacetate (0.142 g, 1.25 mmol, Tokyo Chemical Industry) were added. After stirring for 21 hours, water (20 mL) was added, followed by extraction with ethyl acetate (30 mL, twice, Kishida Chemical). The recovered ethyl acetate solution was washed with a saturated saline solution (10 mL), followed by adding sodium sulfate (Kishida Chemical) and filtration. The filtrate was distilled off under reduced pressure, and the residue was subjected to silica gel column chromatography (Kanto Chemical), to give Compound 15. Yield amount: 0.140 g, yield: 70%.

### 3-5) Synthesis of ImdM-SS

Compound 15 (0.911 g, 3.55 mmol) was dissolved in dehydrated methanol (10 mL, Kanto Chemical) at room temperature under a nitrogen atmosphere, followed by adding potassium carbonate (1.00 g, 7.24 mmol, Kishida Chemical). After stirring for 3 hours, the solvent was distilled off under reduced pressure, and water (10 mL) was added, followed by neutralization with hydrochloric acid (1 M). The resultant was extracted with a mixed solvent of chloroform (Kishida Chemical) and 1-propanol (Kishida Chemical) (3:1, 25 mL, 4 times). The solvent was distilled off under reduced pressure, and the obtained residue was purified by reversed-phase high performance liquid chromatography (apparatus: PU-4086 and UV-4075 manufactured by JASCO, column: TA12S05-2520WX), to give ImdM-SS. Yield amount: 27 mg, yield: 4%.

### 3-6) Synthesis of ImdM-SH

ImdM-SS (0.105 g, 0.464 mmol) was dissolved in water (7 mL) at room temperature under a nitrogen atmosphere, followed by adding dithiothreitol (123 mg, 0.797 mmol, NACALAI TESQUE). After stirring for 7 hours, a mixed solvent of chloroform (Kishida Chemical) and 1-propanol (Kishida Chemical) (9:1, 25 mL, 4 times) was added, followed by extraction. Sodium sulfate was added, followed by filtration, the filtrate was distilled off under reduced pressure, and the obtained residue was purified by reversed-phase high performance liquid chromatography (apparatus: PU-4086 and UV-4075 manufactured by JASCO, column: TA12S05-2520WX), to give ImdM-SH. Yield amount: 38 mg, yield: 18%.

### 4) Syntheses of pPyM-SH (para-pyridin-4-ylmethanethiol) and pPyM-SS

The compounds (corresponding to the compounds represented by the formulas (4) and (11), respectively) were synthesized by the following scheme.

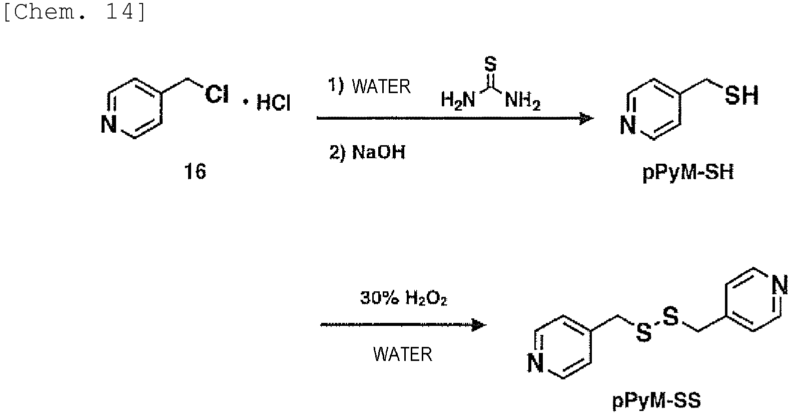

### 4-1) Synthesis of pPyM-SH

Compound 16 (2.175 g, 13.26 mmol) was dissolved in water, followed by adding thiourea (1.450 g, 19.04 mmol, Kishida Chemical). After heating to 90°C, the obtained mixture was stirred for 1.5 hours and cooled to room temperature. Sodium hydroxide (2.058 g, 51.45 mmol, Kishida Chemical) was added, and the obtained mixture was stirred for 22 hours, followed by washing with tert-butyl methyl ether (30 mL, twice, Kanto Chemical). The obtained aqueous solution was neutralized with hydrochloric acid (1 M, Kishida Chemical), followed by extraction with chloroform (30 mL, Kishida Chemical). Sodium sulfate (Kishida Chemical) was added, followed by filtration. The filtrate was distilled off under reduced pressure, and the obtained residue was purified by silica gel column chromatography (Kanto Chemical), to give pPyM-SH. Yield amount: 459 mg, yield: 14%.

### 4-2) Synthesis of pPyM-SS

pPyM-SH (51 mg, 0.41 mmol) was dissolved in water (1 mL), followed by adding a 30% hydrogen peroxide solution (1 mL, Kishida Chemical). After stirring for 14 hours, the obtained mixture was extracted with methylene chloride (10 mL, 3 times, Kishida Chemical). Sodium sulfate was added, followed by filtration. The filtrate was distilled off under reduced pressure to give pPyM-SS. Yield amount: 46 mg, yield: 91%.

### 5) Syntheses of oPyM-SH (ortho-pyridin-2-ylmethanethiol) and oPyM-SS

The compounds (corresponding to the compounds represented by the formulas (5) and (12), respectively) were synthesized by the following scheme.

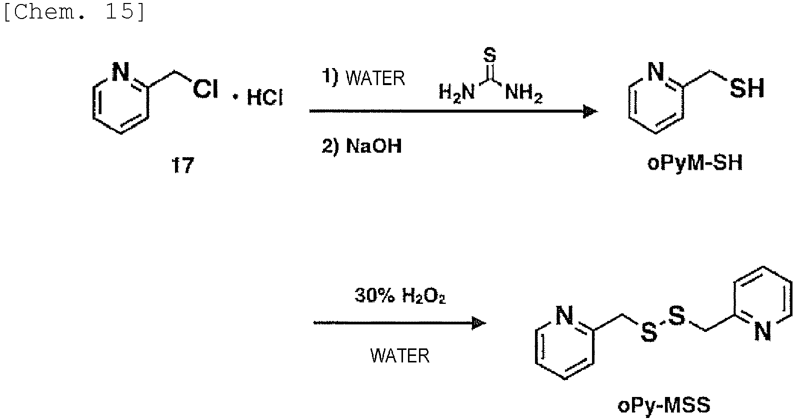

### 5-1) Synthesis of oPyM-SH

Compound 17 (1.035 g, 6.309 mmol, Tokyo Chemical Industry) was dissolved in water, followed by adding thiourea (0.713 g, 9.36 mmol, Kishida Chemical). After heating to 90°C, the obtained mixture was stirred for 1 hour and cooled to room temperature. Sodium hydroxide (1.00 g, 25.0 mmol, Kishida Chemical) was added, and the obtained mixture was stirred for 9 hours, followed by washing with tert-butyl methyl ether (40 mL, Kanto Chemical). The obtained aqueous solution was neutralized with hydrochloric acid (1 M, Kishida Chemical), followed by extraction with methylene chloride (30 mL, 3 times, Kishida Chemical). To the recovered methylene chloride solution was added sodium sulfate (Kishida Chemical), followed by filtration. The filtrate was distilled off under reduced pressure, and the obtained residue (an oPyM-SH crude product) was purified by high performance liquid chromatography (apparatus: PU-4086 and UV-4075 manufactured by JASCO, column: TA12S05-2520WX), to give oPyM-SH. Yield amount: 113 mg, yield: 8%.

### 5-2) Synthesis of oPyM-SS

The oPyM-SH crude product obtained by the above method was dissolved in a 30% hydrogen peroxide solution (8 mL, Kishida Chemical), followed by stirring at room temperature for 1 hour. The obtained mixture was extracted with chloroform (30 mL, 3 times, Kishida Chemical), and to the recovered chloroform solution was added sodium sulfate (Kishida Chemical), followed by filtration. The filtrate was distilled off under reduced pressure, and the obtained residue was purified by silica gel column chromatography (FUJI SILYSIA CHEMICAL), to give oPyM-SS. Yield amount: 916 mg, yield: 58%.

### 6) Syntheses of pPyM-SH-NMe·M ((4-(mercaptomethyl)-1-methylpyridin-1-ium) halide) and pPyM-SS-NMe·M

The compounds (corresponding to the compounds represented by the formulas (6) and (13), respectively) were synthesized by the following scheme.

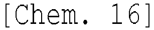

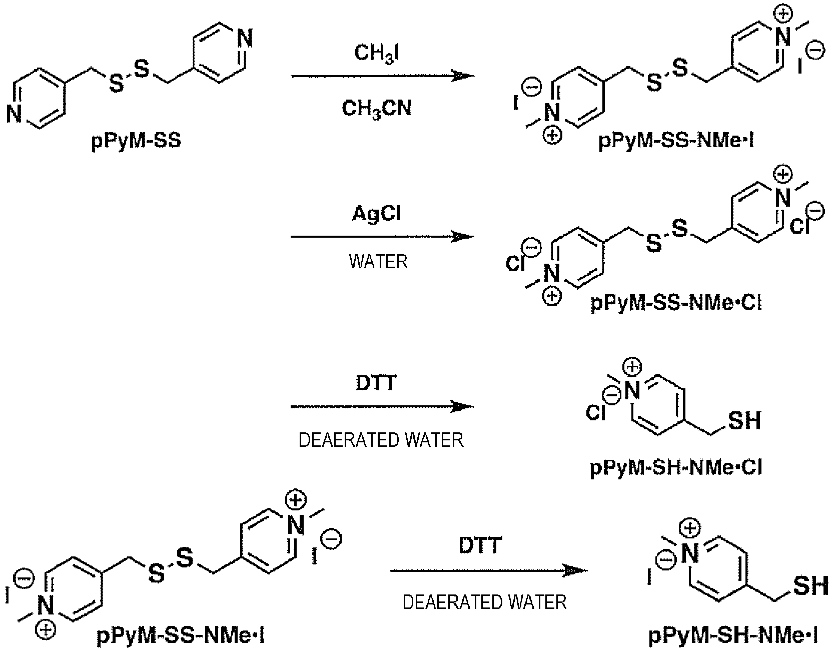

### 6-1) Synthesis of pPyM-SS-NMe·I (4,4'-(disulfanediylbis(methylene))bis(1-methylpyridin-1-ium) diiodide)

pPyM-SS (254 mg, 1.02 mmol) obtained by the above method was dissolved in acetonitrile (3.0 mL, Kishida Chemical) and acetone (3 mL, Kishida Chemical), followed by adding iodomethane (1.503 g, 10.59 mmol, Tokyo Chemical). After heating to 60°C, the obtained mixture was stirred for 16 hours and cooled to room temperature. The resulting precipitate was filtered, to give pPyM-SS-NMe·I from the obtained residue. Yield amount: 446 mg, yield: 82%.

### 6-2) Synthesis of pPyM-SS-NMe·Cl (4,4'-(disulfanediylbis(methylene))bis(1-methylpyridin-1-ium) dichloride)

pPyM-SS-NMe·I (192 mg, 0.361 mmol) was dissolved in water (3.5 mL), followed by adding silver chloride (I) (115 mg, 0.805 mmol, Kishida Chemical). After stirring at room temperature for 4 hours in a dark place, the resulting precipitate was filtered. The filtrate was distilled off under reduced pressure, followed by adding ethanol (2 mL, Kishida Chemical), and the resulting precipitate was filtered. The filtrate was distilled off under reduced pressure, to give pPyM-SS-NMe·Cl. Yield amount: 80 mg, yield: 63%.

### 6-3) Synthesis of pPyM-SH-NMe·Cl ((4-(mercaptomethyl)-1-methylpyridin-1-ium) chloride)

pPyM-SS-NMe·Cl (39 mg, 0.141 mmol) was dissolved in deaerated water (2.1 mL), followed by adding dithiothreitol (DTT, 29 mg, 0.187 mmol, NACALAI TESQUE). The obtained mixture was stirred at room temperature for 6 hours, then distilled off under reduced pressure, and the obtained residue (a pPyM-SH-NMe·Cl crude product) was purified by high performance liquid chromatography (apparatus: PU-4086 and UV-4075 manufactured by JASCO, column: TA12S05-2520WX), to give pPyM-SH-NMe·Cl. Yield amount: 40 mg, yield: 81%.

### 6-4) Synthesis of pPyM-SH-NMe·I ((4-(mercaptomethyl)-1-methylpyridin-1-ium) iodide)

pPyM-SS-NMe·I (104 mg, 0.195 mmol) obtained by the above method was dissolved in deaerated water (3.1 mL), followed by adding dithiothreitol (DTT, 125 mg, 0.809 mmol, NACALAI TESQUE). After stirring at room temperature for 3 hours, 2 M hydrochloric acid (1 mL, Kishida Chemical) was added, followed by distilling off under reduced pressure, and the obtained residue (a pPyM-SH-NMe·I crude product) was purified by high performance liquid chromatography (apparatus: PU-4086 and UV-4075 manufactured by JASCO, column: TA12S05-2520WX), to obtain pPyM-SH-NMe·I. Yield amount: 23 mg, yield: 22%.

### 7) Synthesis of oPyM-SH-NMe·M ((2-(mercaptomethyl)-1-methylpyridin-1-ium) halide) and oPyM-SS-NMe·M (2,2'-(disulfanediylbis(methylene))bis(1-methylpyridin-1-ium) halide)

The compounds (corresponding to the compounds represented by the formulas (7) and (14), respectively) were synthesized by the following scheme.

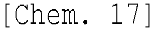

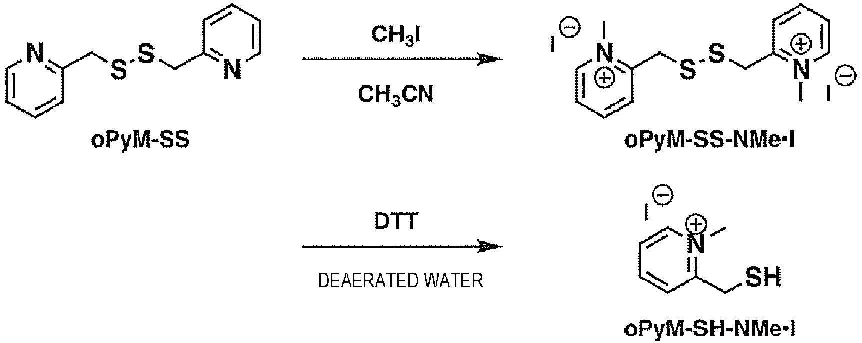

### 7-1) Synthesis of oPyM-SS-NMe·I (2,2'-(disulfanediylbis(methylene))bis(1-methylpyridin-1-ium) diiodide)

oPyM-SS (739 mg, 2.97 mmol) obtained by the above method was dissolved in acetonitrile (9.7 mL, Kishida Chemical) and acetone (10 mL, Kishida Chemical), followed by adding iodomethane (4.434 g, 31.24 mmol, Tokyo Chemical). After heating to 65°C, the obtained mixture was stirred for 16 hours and cooled to room temperature. The resulting precipitate was filtered, to give oPyM-SS-NMe·I from the obtained residue. Yield amount: 1.286 g, and yield: 81%.

### 7-2) Synthesis of oPyM-SH-NMe·I ((2-(mercaptomethyl)-1-methylpyridin-1-ium) iodide)

oPyM-SS-NMe·I (108 mg, 0.203 mmol) was dissolved in deaerated water (3 mL), followed by adding dithiothreitol (DTT, 130 mg, 0.840 mmol, NACALAI TESQUE). After stirring at room temperature for 5 hours, 2 M hydrochloric acid (1 mL, Kishida Chemical) was added, followed by washing with methylene chloride (20 mL, 5 times, Kishida Chemical). The recovered aqueous solution was distilled off under reduced pressure, and the obtained residue (an oPyM-SH-NMe·I crude product) was purified by high performance liquid chromatography (apparatus: PU-4086 and UV-4075 manufactured by JASCO, column: TA12S05-2520WX), to give oPyM-SH-NMe·I. Yield amount: 80 mg, and yield: 74%.

### 8) Preparation of Reduced and Denatured Protein

A bovine pancreas trypsin inhibitor (BPTI) (see Reference Document 1 and Reference Document 2 below), RNase A (see Reference Document 3 below), and β₂-microglobulin (β2m) were used as model substrates. BPTI and RNase A are proteins having three and four disulfide bonds, respectively, and are generally used as model substrates in the field. Further, β2m was used as a model substrate having one disulfide bond.

In order to prepare a reduced and denatured product of BPTI (Takara Bio Inc.) (a reduced product having three disulfide bonds), 10 mg of BPTI was incubated in presence of 8 M urea and 20 mM DTT at pH 8.0 and 50°C for 3 hours, followed by purification by reversed-phase HPLC. After it was found by MALDI-TOF/MS that all disulfide bonds of the purified sample were cleaved, the sample was freeze-dried and stored at -80°C (see Reference Document 1 below).

In order to prepare a reduced and denatured product of RNase A (SIGMA) (a reduced product having four disulfide bonds), 8 mg of RNase A was incubated in presence of 6 M guanidine hydrochloric acid and 100 mM DTT at pH 8.7 and 25°C for 2 hours, followed by dialysis with 10 mM HCl. Further, dialysis was performed twice, DTT and the like were removed, and the solution was replaced with 10 mM HCl (see Reference Document 3 below).

β2m was expressed as a recombinant by an E. coli expression system, and the collected cells were suspended in lysate A (50 mM Tris-HCl, pH 8.1; 300 mM NaCl), followed by ultrasonic disintegration. The fraction obtained after centrifugation was incubated in the presence of 8 M urea and 20 mM DTT at pH 8.0 and 50°C for 3 hours, followed by purification by reversed-phase column chromatography (Hitachi) using a Cosmosil 5C₁₈-AR-II column.

Reference Document 1: M. Okumura, H. Kadokura, S. Hashimoto, K. Yutani, S. Kanemura, T. Hikima, Y. Hidaka, L. Ito, K. Shiba, S. Masui, D. Imai, S. Imaoka, H. Yamaguchi, K. Inaba, Inhibition of the functional interplay between endoplasmic reticulum (ER) oxidoreduclin-1α (Ero1α) and protein-disulfide isomerase (PDI) by the endocrine disruptor bisphenol A. J Biol Chem. 2014 289(39):27004-27018.

Reference Document 2: J.S. Weissman, P.S. Kim, Reexamination of the folding of BPTI: predominance of native intermediates. Science 1991 253(5026) :1386-93.

Reference Document 3: M.M. Lyles, H.F. Gilbert Catalysis of the oxidative folding of ribonuclease A by protein disulfide isomerase: dependence of the rate on the composition of the redox buffer. Biochemistry. 1991 30(3) :613-9.

### 9) Evaluation of Disulfide Bond Introduction Ability into Reduced and Denatured BPTI

A buffer having the following combinations of 1 mM of a thiol compound and 0.2 mM of a disulfide compound (50 mM Tris-HCl, pH 7.5; 300 mM NaCl) was incubated at 30°C with 30 µM of the reduced and denatured BPTI prepared in the above 8), followed by a folding reaction.

### Comparative Example 1: GSH (nakalai)/GSSG (nakalai)

### Example 1: LDA-SH/LDA-SS

### Example 2: BDA-SH/BDA-SS

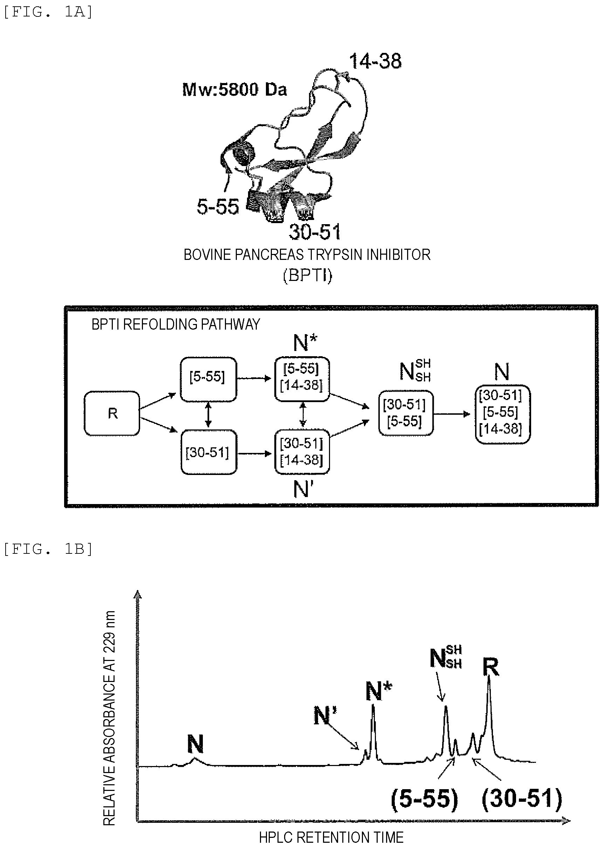

The reaction solution was dispensed over time, followed by adding an equal amount of 1 N HCl, and the thiol group and disulfide bond exchange reaction was quenched. In order to identify the molecular species (see FIG. 1A) in the reaction solution, the resultant was subjected to reversed-phase HPLC. As illustrated in FIG. 1A and FIG. 1B, an analysis using reversed-phase HPLC can separate and identify each disulfide bond species when BPTI is folded, and can track a disulfide bond crosslinking position in a change over time.

Measurement and analysis conditions of the reversed-phase HPLC are shown below.

TSKgel Protein C4-300 column (4.6 × 150 mm; Tosoh Bioscience) was used as a column, and an absorbance at 229 nm was detected. Mixing an aqueous solution containing 0.05% trifluoroacetic acid (solution A) and an acetonitrile solution containing 0.05% trifluoroacetic acid (solution B) was developed at a linear concentration gradient in which a rate of increase in volume of the solution B in the mixed solution was 1%/min from 0 to 15 minutes and 0.5%/min from 15 to 115 minutes.

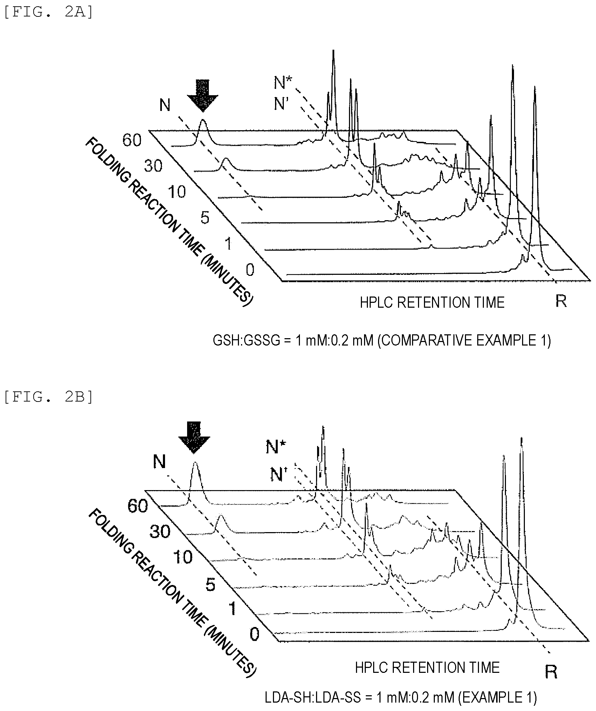

When GSH/GSSG was used (Comparative Example 1), a yield of a native type (N) at a folding reaction time of 60 minutes was 26% (FIG. 2A). Further, when LDA-SH/LDA-SS was used (Example 1), a yield of the native type (N) at a folding reaction time of 60 minutes was 31% (FIG. 2B). This indicates that LDA-SH has a folding promoting effect higher than that of GSH.

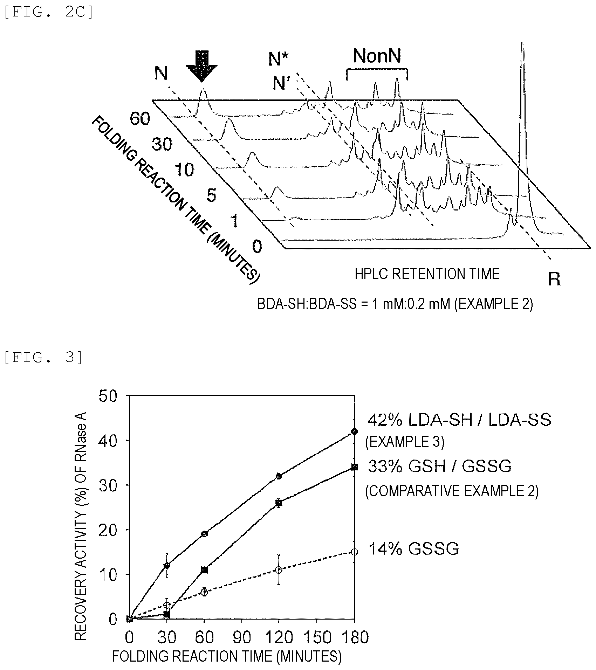

It is found that, when BDA-SH/BDA-SS is used (Example 2), within 1 minute after starting the reaction, R which is a reduced and denatured product of BPTI (all thiol groups) disappears, a native type (N) is generated, and disulfide bonds are extremely rapidly introduced (FIG. 2C). Further, it is found that various types of non-native structural BPTI (shown by NonN in FIG. 2C) are simultaneously generated. In general, a non-native type protein in which a disulfide bond occurs exhibits an aggregation property. However, it is found that since it can be subjected to the measurement by HPLC as described above, BDA-SH/BDA-SS suppresses the aggregation property of non-native type BPTI and has a property as a solubilizing agent.

### 10) RNase A Activity Evaluation 1

In order to evaluate the folding promoting effect, an activity recovery measurement on RNase A prepared in the above 8) was performed. A buffer containing 8 µM RNase A (50 mM Tris-HCl, pH 7.5; 300 mM NaCl) was incubated at 30°C in presence of the following combinations of 1 mM of a thiol compound and 0.2 mM of a disulfide compound.

### Comparative Example 2: GSH/GSSG

### Example 3: LDA-SH/LDA-SS

The reaction solution was dispensed over time, and diluted with the same buffer containing cytidine 2':3'-cyclic monophosphate (cCMP) serving as a substrate of RNase A, to obtain a final concentration of 4 µM RNase A and 0.4 mM cCMP. A change in absorbance at 284 nm was measured using a spectrophotometer, and a nucleic acid degradation activity of RNase A folded into a native structure was quantified.

As illustrated in FIG. 3, a recovery activity of RNase A after incubation for 3 hours was 42% in presence of LDA-SH/LDA-SS (Example 3), which was higher than that in a case of 0.2 mM GSSG (14%) and in a case of GSH/GSSG (Comparative Example 2) (33%). This indicates that LDA-SH/LDA-SS has the folding promoting effect.

### 11) Evaluation of Disulfide Bond Introduction Ability into Reduced and Denatured β2m

A buffer having the following combinations of 1 mM of a thiol compound and 0.2 mM of a disulfide compound (50 mM Tris-HCl, pH 7.5; 300 mM NaCl) was incubated at 30°C with 10 µM of the reduced and denatured β2m prepared in the above 8), followed by a folding reaction.

### Comparative Example 3: GSH/GSSG

### Example 4: LDA-SH/LDA-SS

### Example 5: BDA-SH/BDA-SS

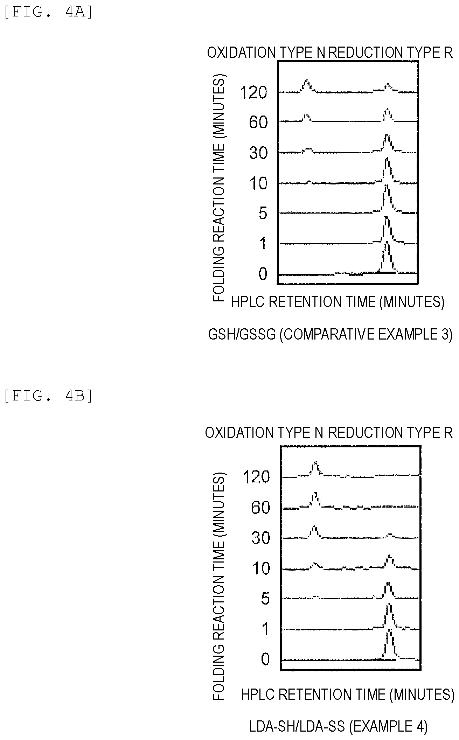

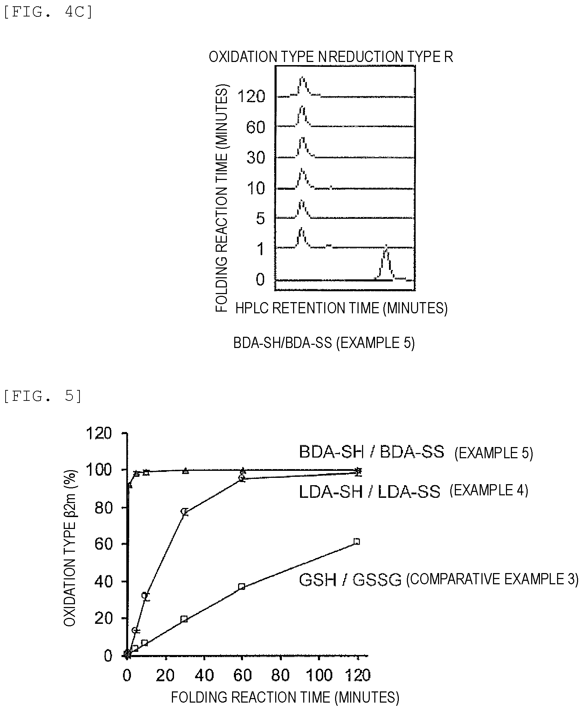

The reaction solution was dispensed over time, followed by adding an equal amount of 6 M guanidine hydrochloric acid and 1.5 M HCl, and the disulfide bond introduction reaction was quenched. In order to identify the molecular species in the reaction solution, the resultant was subjected to reversed-phase HPLC, and an oxidation type (N) and a reduction type (R) of β2m were analyzed (FIG. 4A to FIG. 4C and FIG. 5). The molecular species of the oxidation type (N) and the reduction type (R) were identified by MALDI-TOF/MS.

Measurement and analysis conditions of the reversed-phase HPLC are shown below. 5C18-AR2 column (4.6 × 150 mm; NACALAI TESQUE) was used as a column, and an absorbance at 229 nm was detected. Mixing of an aqueous solution containing 0.1% trifluoroacetic acid (solution A) and an acetonitrile solution containing 0.1% trifluoroacetic acid (solution B) was developed at a linear concentration gradient in which a rate of increase in volume of the solution B in the mixed solution was 1%/min from 0 to 10 minutes and 0.5%/min from 10 to 35 minutes.

From FIG. 4A to FIG. 4C and FIG. 5, when GSH/GSSG was used (Comparative Example 3), a yield at a folding reaction time of 60 minutes was 36%. When LDA-SH/LDA-SS was used (Example 4), a yield at a folding reaction time of 60 minutes was 95%. When BDA-SH/BDA-SS was used (Example 5), a yield at a folding reaction time of 60 minutes was 100%.

Based on these results, when a reaction rate constant was calculated by performing the disulfide bond introduction reaction to the reduced and denatured β2m as a pseudo-first-order reaction, GSSG: 1.12 × 10⁻⁴, LDA-SS: 6.96 × 10⁻⁴, and BDA-SS: 3.59 × 10⁻² were obtained. This indicates that a disulfide bond introduction ability of the LDA-SH/LDA-SS (Example 4) is 6.2 times and BDA-SH/BDDA-SS (Example 5) is about 320 times higher than that of GSH/GSSG (Comparative Example 3). β2m is one of ingredients involved in immunity, and a misfolded form thereof is associated with a pathology of dialysis amyloidosis. The disulfide bond introduction agent according to the invention capable of introducing disulfide bonds at a high speed into β2m, which is a light chain of a major histocompatibility complex responsible for a basis of an immune system, is useful in terms of efficient production of a protein preparation.

### 12) RNase A Activity Evaluation 2

In order to evaluate the folding promoting effect, an activity recovery measurement of RNase A prepared in the above 8) was performed. A buffer containing 8 µM RNase A (50 mM Tris-HCl, pH 7.5; 300 mM NaCl) was incubated at 30°C in presence of the following combinations of 1 mM of a thiol compound and 0.2 mM of a disulfide compound.

### Comparative Example 4: GSH/GSSG

### Example 6: ImdM-SH/ImdM-SS

### Example 7: oPyM-SH/oPyM-SS

The reaction solution was dispensed over time, and diluted with the same buffer containing cytidine 2':3'-cyclic monophosphate (cCMP) serving as a substrate of RNase A, to obtain a final concentration of 4 µM RNase A and 0.4 mM cCMP. A change in absorbance at 284 nm was measured using a spectrophotometer, and a nucleic acid degradation activity of RNase A folded into a native structure was quantified.

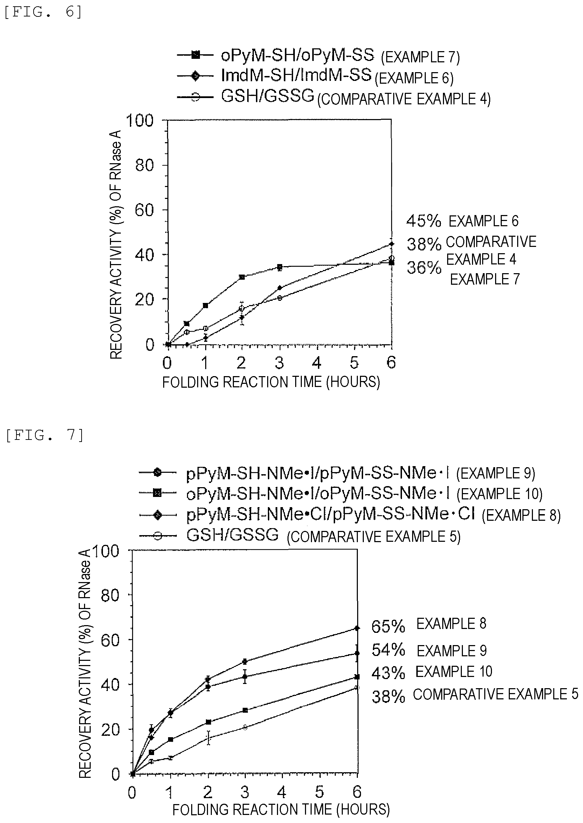

As illustrated in FIG. 6, a recovery activity of RNase A after incubation for 6 hours was 45% in presence of ImdM-SH/ImdM-SS (Example 6), which was higher than that in a case of GSH/GSSG (Comparative Example 4) (38%). This indicates that ImdM-SH/ImdM-SS has the folding promoting effect. Further, it indicates that a recovery activity of RNase A after incubation for 3 hours in a case of oPyM-SH/oPyM-SS (Example 7) is higher than that in a case of GSH/GSSG (Comparative Example 4), and the folding promoting effect is high in an initial stage up to 3 hours.

### 13) RNase A Activity Evaluation 3

In order to evaluate the folding promoting effect, an activity recovery measurement of RNase A prepared in the above 8) was performed. A buffer containing 8 µM RNase A (50 mM Tris-HCl, pH 7.5; 300 mM NaCl) was incubated at 30°C in presence of the following combinations of 1 mM of a thiol compound and 0.2 mM of a disulfide compound.

### Comparative Example 5: GSH/GSSG

### Example 8: pPyM-SH-NMe-Cl/pPyM-SS-NMe-Cl

### Example 9: pPyM-SH-NMe·I/pPyM-SS-NMe·I

### Example 10: oPyM-SH-NMe·I/oPyM-SS-NMe·I

The reaction solution was dispensed over time, and diluted with the same buffer containing cytidine 2':3'-cyclic monophosphate (cCMP) serving as a substrate of RNase A, to obtain a final concentration of 4 µM RNase A and 0.4 mM cCMP. A change in absorbance at 284 nm was measured using a spectrophotometer, and a nucleic acid degradation activity of RNase A folded into a native structure was quantified.

As illustrated in FIG. 7, a recovery activity of RNase A after incubation for 6 hours was 65% in presence of pPyM-SH-NMe-Cl/pPyM-SS-NMe·Cl (Example 8), 54% in presence of pPyM-SH-NMe·I/pPyM-SS-NMe·I (Example 9), and 43% in presence of oPyM-SH-NMe·I/oPyM-SS-NMe·I (Example 10), which were higher than that in a case of GSH/GSSG (Comparative Example 5) (38%). This indicates that pPyM-SH-NMe·Cl/pPyM-SS-NMe·Cl, pPyM-SH-NMe·I/pPyM-SS-NMe·I, and oPyM-SH-NMe·I/oPyM-SS-NMe·I have the folding promoting effect.

### 14) β2m Aggregation Experiment by Heating

### (No Additive)

A buffer containing 10 µM of native type β2m (50 mM Tris-HCl, pH 7.5; 300 mM NaCl) was heated from 25°C to 50°C, equilibrated for 10 minutes when the temperature reached 50°C, and then cooled to 25°C. For a supernatant liquid of the fraction obtained after centrifugation, a residual rate of the native type β2m in the supernatant liquid was measured by measuring an absorbance at 280 nm using a spectrophotometer.

### (With Additive)

For a buffer containing 10 µM of native type β2m (50 mM Tris-HCl, pH 7.5; 300 mM NaCl), 1 mM of the following thiol compound was added, and the obtained mixture was heated from 25°C to 50°C, equilibrated for 10 minutes when the temperature reached 50°C, and then cooled to 25°C.

### Comparative Example 6: GSH

### Example 11: LDA-SH

### Example 12: BDA-SH

For a supernatant liquid of the fraction obtained after centrifugation, a residual rate of the native type β2m in the supernatant liquid was measured by measuring an absorbance at 280 nm using a spectrophotometer.

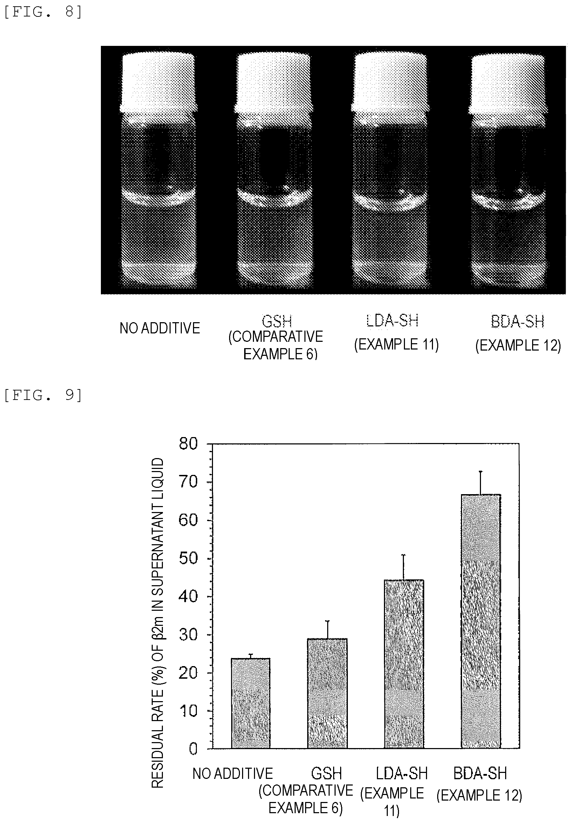

FIG. 8 illustrates a photograph of a solution after cooling (before centrifugation). From FIG. 8, it is found that in LDA-SH (Example 11) and BDA-SH (Example 12), the solution is transparent and the aggregation of the native type β2m is suppressed as compared with that in a case of no additive and in a case of GSH (Comparative Example 6) . Further, results of measuring the residual rate of native type β2m in the supernatant liquid obtained by centrifugation are illustrated in FIG. 9. From FIG. 9, it is found that in LDA-SH (Example 11) and BDA-SH (Example 12), the residual rate of the native type β2m is higher than that in the case of no additive and in the case of GSH (Comparative Example 6), and therefore, LDA-SH and BDA-SH have a high function of suppressing the aggregation of proteins and a high solubilizing function.

### 15) RNase A Activity Evaluation 4

In order to evaluate the folding promoting effect, an activity recovery measurement of RNase A prepared in the above 8) was performed. A buffer containing 8 µM RNase A (50 mM Tris-HCl, pH 7.5; 300 mM NaCl) was incubated at 30°C in presence of the following combination of 1 mM of a thiol compound and 0.2 mM of a disulfide compound.

### Example 13: pPyM-SH/GSSG

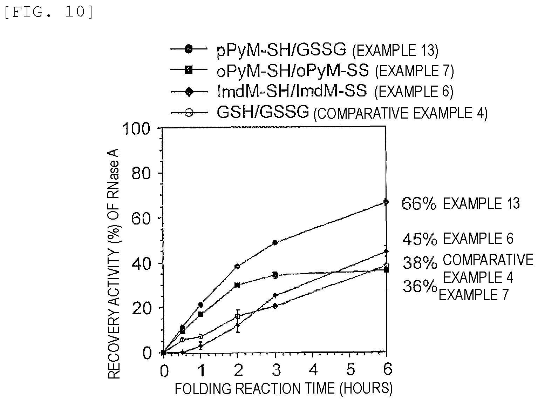

The reaction solution was dispensed over time, and diluted with the same buffer containing cytidine 2':3'-cyclic monophosphate (cCMP) serving as a substrate of RNase A, to obtain a final concentration of 4 µM RNase A and 0.4 mM cCMP. A change in absorbance at 284 nm was measured using a spectrophotometer, and a nucleic acid degradation activity of RNase A folded into a native structure was quantified. Results are illustrated in FIG. 10 together with Comparative Example 4, Example 6, and Example 7 in "the above 12) RNase A Activity Evaluation 2".

### Industrial Applicability

The protein folding agent according to the invention can be used as an oxidative folding accelerator for protein preparations such as an antibody having many disulfide bonds. The protein folding agent according to the invention can be used as a high-speed oxidative folding accelerator for a protein having a single disulfide bond. Further, the protein folding agent according to the invention can be used as a reagent artificially accumulating misfolded proteins in a soluble state and as a research reagent for basic research in the biochemical field.

All publications, patents, and patent applications cited in the present description are incorporated in the present description by reference as they are.

## Claims

1. A protein folding agent comprising:
as an active ingredient, at least one selected from compounds represented by the following formulas, salts thereof, and solvates thereof, 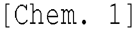 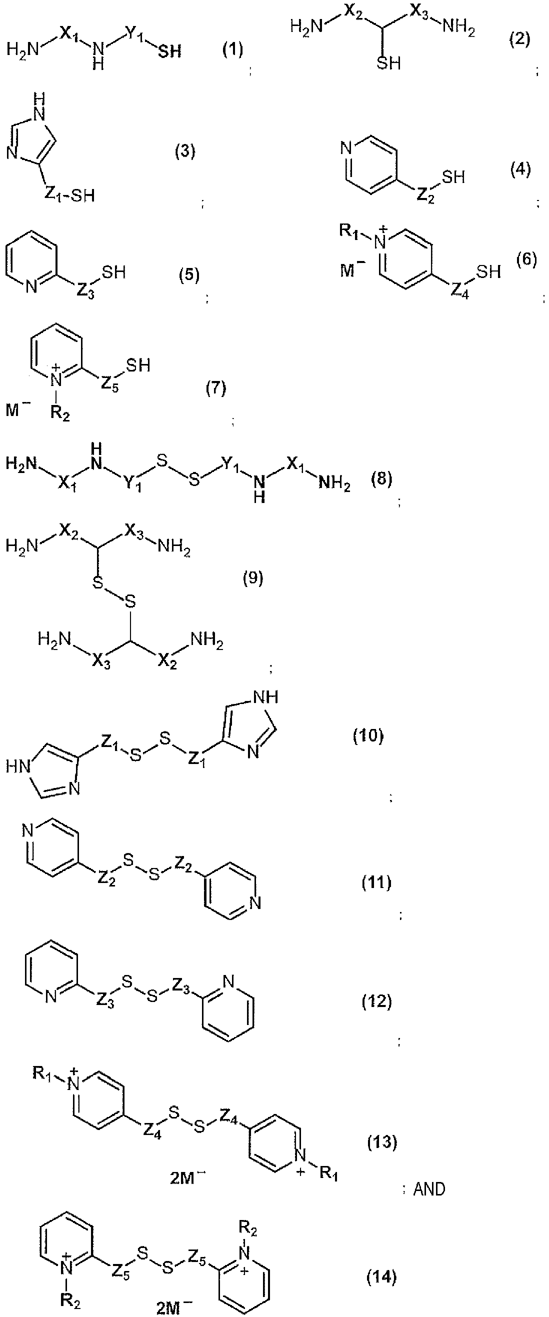 [in the formulas,
X₁, X₂, and X₃ are each independently an alkylene group having 1 to 10 carbon atoms which may contain 1 to 5 oxygen atoms in a molecular chain,
Y₁'s are each independently an alkylene group having 1 to 10 carbon atoms which may contain 1 to 5 oxygen atoms in a molecular chain,
Z₁, Z₂, Z₃, Z₄, and Z₅ are each independently an alkylene group having 1 to 10 carbon atoms which may contain 1 to 5 oxygen atoms in a molecular chain,
R₁ and R₂ are each independently an alkyl group having 1 to 24 carbon atoms, and
M⁻'s are each independently a chlorine ion, a bromine ion, or an iodide ion].

2. The protein folding agent according to claim 1, wherein
at least one selected from the compounds represented by the formulas (1) to (7), the salts thereof, and the solvates thereof is contained as the active ingredient.

3. The protein folding agent according to claim 1, wherein
at least one selected from the compounds represented by the formulas (8) to (14), the salts thereof, and the solvates thereof is contained as the active ingredient.

4. The protein folding agent according to claim 1, wherein
at least one selected from: the compounds represented by formulas (1) to (7), the salts thereof, and the solvates thereof; and at least one selected from the compounds represented by the formulas (8) to (14), the salts thereof, and the solvates thereof is contained as the active ingredient.

5. A protein folding method comprising:
treating an unfolded protein in presence of at least one selected from the compounds represented by the formulas (1) to (14) defined in claim 1, the salts thereof, and the solvates thereof.

6. A protein renaturation method comprising:
treating an unfolded protein in presence of at least one selected from the compounds represented by the formulas (1) to (14) defined in claim 1, the salts thereof, and the solvates thereof.

7. A protein solubilizing agent comprising:
as an active ingredient, at least one selected from the compounds represented by the formulas (1) to (14) defined in claim 1, the salts thereof, and the solvates thereof.
